# EUROPEAN PATENT APPLICATION

(11) **EP 4 814 121 A1**
(43) Date of publication of application: **30.09.2026**
(21) Application number: 24894642.8
(22) Date of filing: 21.11.2024
(51) Int. Cl.: C12N 15/82, C12N 15/113, C12N 9/22, C12N 15/10

(54) **METHOD FOR INCREASING PRIME EDITING EFFICIENCY IN PLANT**

(30) Priority: 21.11.2023 KR 20230161962
(71) Applicant: Nulla Bio Inc., Jinju-si, Gyeongsangnam-do 52828 (KR)
(72) Inventor: KIM, Jae Yean, Jinju-si Gyeongsangnam-do 52826 (KR); VU, Tien Van, Jinju-si Gyeongsangnam-do 52829 (KR); NGUYEN, Thi Ngan, Jinju-si Gyeongsangnam-do 52829 (KR); KIM, Jihae, Miryang-si Gyeongsangnam-do 50429 (KR); NGUYEN, Hoai Thu, Jinju-si Gyeongsangnam-do 52828 (KR); SONG, Young Jong, Jinju-si Gyeongsangnam-do 52693 (KR)
(74) Representative: Gulde & Partner
(86) International application number: PCT/KR2024/018501
(87) International publication number: WO 2025/110750

(57) **Abstract**

The present invention relates to a method for enhancing prime editing efficiency in plants, and more specifically, to a recombinant vector for prime editing in plants with enhanced editing efficiency, which comprises: a virus-based replicon including a prime editing guide RNA (pegRNA) expression cassette regulated by a U6 composite promoter; and a prime editor expression cassette, and a use thereof.

## Description

### TECHNICAL FIELD

The present invention relates to a method for enhancing prime editing efficiency in plants.

### BACKGROUND ART

Prime editing is a CRISPR-Cas9-based tool first described in 2019 by Anzalone et al. (Nature 2019, 576, 149-157). Prime editing does not induce a double-strand break (DSB) and can precisely correct all types of point mutations, insertions, and deletions; accordingly, it has been reported to have the potential to correct approximately 89% of human pathogenic genetic variants.

The prime editing technology requires two main components. The first is a prime editor (PE) protein composed of a reverse transcriptase fused to a nickase Cas9 (nCas9). The second is a prime editing guide RNA (pegRNA), which guides the PE to a target site and functions as a template for the desired correction. The pegRNA comprises a primer binding site (PBS), which serves as a docking site for the reverse transcriptase, and a reverse transcription template (RTT) containing the desired edit information.

Following the first report of a PE by Anzalone et al., improvements and applications of PE have been reported in various organisms. Since the initial PE components, all elements have recently been modified and endowed with new properties, resulting in increased efficiency of prime editing. However, the application of PE in plants has shown inconsistencies among different loci and plant species, and in particular, considerable efforts are still required to improve the efficiency of prime editing in dicotyledonous (dicot) plants.

Accordingly, the present invention is intended to provide a method for enhancing prime editing efficiency in plants, particularly in dicotyledonous plants.

Meanwhile, Korean Patent Publication No. 2022-0112698 discloses a "composition for prime editing with improved editing efficiency," and Korean Patent Publication No. 2023-0075420 discloses "prime editing using an HIV reverse transcriptase and Cas9 or a variant thereof"; however, neither document discloses the "method for enhancing prime editing efficiency in plants" of the present invention.

### DETAILED DESCRIPTION

### TECHNICAL PROBLEMS TO BE SOLVED

In order to enhance prime editing efficiency in plants, the present invention provides a method for enhancing prime editing efficiency in plants by experimentally evaluating various conditions, including combinations of the prime editor constituent proteins, types of the prime editor constituent proteins and/or transcriptional regulatory elements (promoters and terminators) for pegRNA, as well as delivery systems, and by presenting optimized combinations thereof.

### TECHNICAL MEANS FOR SOLVING PROBLEMS

In order to solve the above problem, the present invention provides a recombinant vector for plant prime editing with enhanced editing efficiency, comprising a virus-based replicon, wherein the virus-based replicon comprises: a prime editing guide RNA (pegRNA) expression cassette regulated by a U6 composite promoter; and a prime editor expression cassette.

The present invention further provides a method for enhancing prime editing efficiency in plants, comprising transforming one or more plant cells with the aforementioned recombinant vector.

The present invention still further provides a composition for enhancing prime editing efficiency in plants comprising the aforementioned recombinant vector, as an active ingredient.

### ADVANTAGEOUS EFFECT OF THE INVENTION

By using the method according to the present invention, prime editing efficiency in plants, particularly in dicotyledonous plants, can be improved. Therefore, the method of the present invention may be usefully applied in the field of new variety breeding based on precise gene editing technology

### BRIEF DESCRIPTION OF DRAWINGS

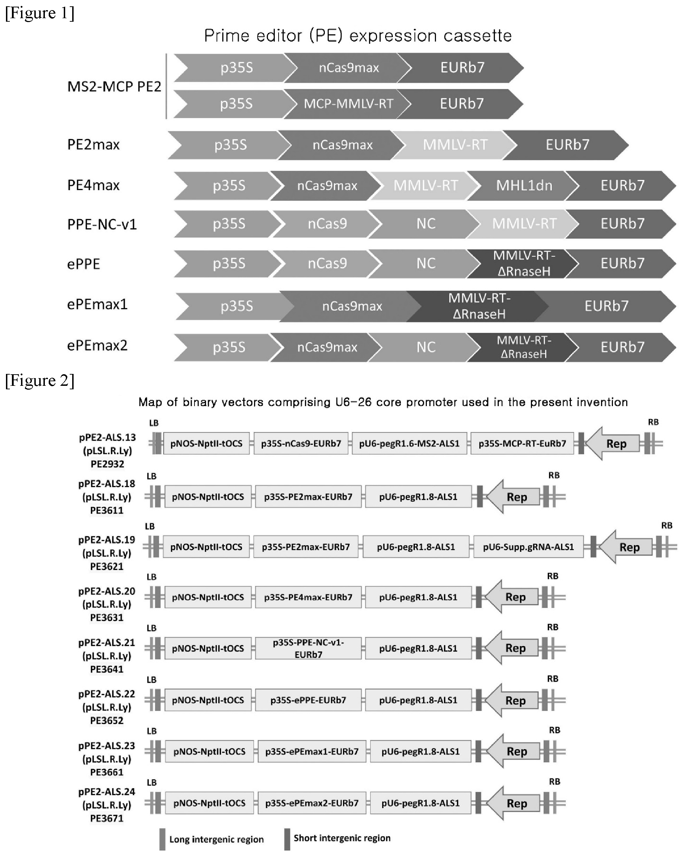

Figure 1 is a schematic diagram of a prime editor (PE) expression cassette used in the present invention. p35S: CaMV 35S promoter, nCas9max: mutant SpCas9 (R221K, N394K, D840A), nCas9: mutant SpCas9 (D840A), MMLV-RT: mutant MMLV reverse transcriptase (D200N, T306K, W313F, T330P, L603W), MMLV-RT-△RnaseH: RNaseH domain truncated mutant MMLV reverse transcriptase (D200N, T306K, W313F, T330P, L603W), MLH1dn: truncated MLH1 D754-756 lacking endonuclease domain, NC; viral nucleocapsid protein, EURb7: EU + Rb7 dual terminator, MCP-MMLV-RT: MS2 coat protein fused MMLV-RT (MCP was cloned from pREDIT_MCP-RecT, MMLV RT is a tobacco codon-optimized MMLV RT from the PE2).

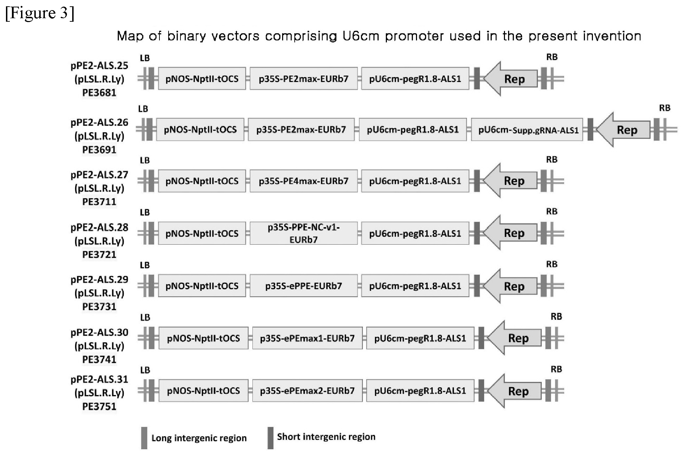

Figures 2 and 3 are schematic diagrams of recombinant vectors for prime editing used in the Examples of the present invention. Figure 2 shows a vector in which expression of pegRNA is regulated by a U6-26 core promoter, and Figure 3 shows a vector in which expression of pegRNA is regulated by a U6 composite promoter. Supp.gRNA refers to a gRNA that assists the prime editing reaction by binding near the pegRNA for *ALS1* prime editing, and consists of the nucleotide sequence of SEQ ID NO: 258. pegR1.6-MS2 consists of the nucleotide sequence of SEQ ID NO: 257. pegR1.8 is formed by linking the nucleotide sequences of gRNA and RTT+PBS (Table 1) to both ends of the gRNA scaffold sequence of Cas9 (corresponding to nucleotides 92 to 177 of SEQ ID NO: 258).
Figure 4 shows the results of analyzing prime editing correction efficiency using the recombinant vectors of Figures 2 and 3. *: p < 0.05, ***: p < 0.0002, ****: p < 0.0001.

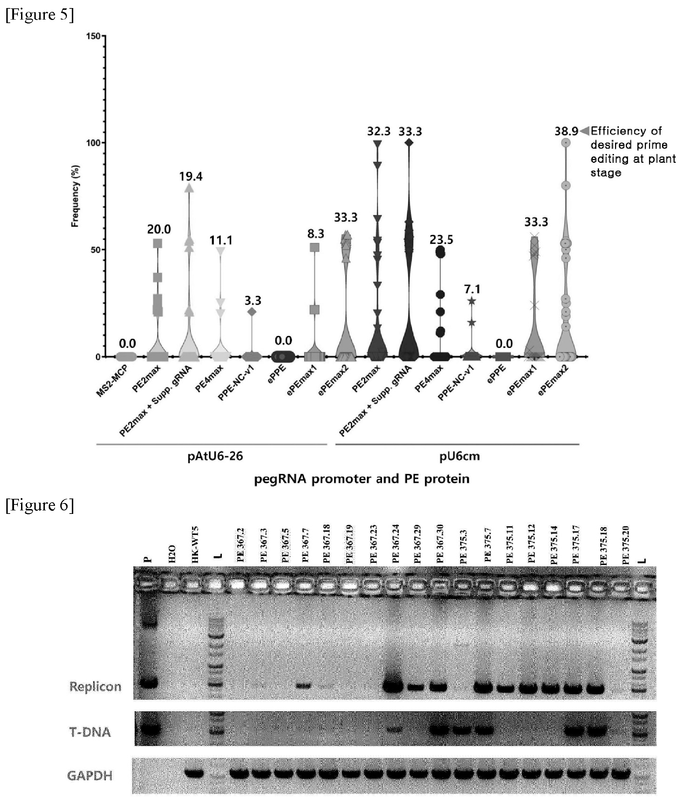

Figure 5 shows the results of analyzing the frequency and efficiency of desired prime editing in regenerated plants. In the graph, the y-axis values represent the frequency (%) of prime editing at the target site analyzed by Sanger sequencing in transformants (i.e., plants regenerated from cotyledon explants transformed with the vector of Figure 2 or Figure 3, corresponding to PE0 events), and the values indicated at the top of each plot represent the efficiency of desired prime editing at the plant stage, calculated by dividing the number of transformants exhibiting desired prime editing by the total number of analyzed transformants.
Figure 6 shows PCR results for determining the presence or absence of replicons and T-DNA in some PE0 event plants.
Figure 7 is a schematic diagram of recombinant vectors used for comparing prime editing efficiency according to different Agrobacterium strains.
Figure 8 shows the results of analyzing prime editing efficiency according to Agrobacterium strains. *: p < 0.05.
Figure 9 shows the results of analyzing the frequency and efficiency of desired prime editing in regenerated plants in a prime editing experiment according to Agrobacterium strains.
Figure 10 is a schematic diagram of recombinant vectors used for comparing prime editing efficiency between T-DNA-based and replicon-based recombinant vectors. pEL2B-3: Golden Gate endlinker (position 3).
Figure 11 shows the results of analyzing prime editing efficiency between T-DNA-based and replicon-based recombinant vectors. ***: p < 0.0002, ****: p < 0.0001.
Figure 12 is a schematic diagram of recombinant vectors used for comparing prime editing efficiency according to temperature conditions during prime editing.
Figure 13 shows the results of analyzing prime editing efficiency according to temperature conditions during prime editing. **: p < 0.0021, ***: p < 0.0002, ****: p < 0.0001.
Figure 14 shows the results of analyzing the frequency and efficiency of desired prime editing in regenerated plants in a prime editing experiment according to temperature conditions during prime editing.
Figure 15 is a schematic diagram of recombinant vectors used for comparing prime editing efficiency at various target positions and loci. npegRNA consists of tRNA(Gly)-target gene gRNA-modified (F+E) SpCas9 gRNA scaffold-RTT of the target gene-tevopreQ1-HDV sequence, and is composed of the nucleotide sequence of SEQ ID NO: 259.
Figure 16 shows the results of analyzing prime editing efficiency at various target positions and loci in tomato plants.
Figure 17 is a schematic diagram of a prime editor expression cassette comprising different terminators.
Figure 18 is a schematic diagram of recombinant vectors used for comparing prime editing efficiency according to the type of terminator in the prime editor expression cassette.
Figure 19 shows the results of analyzing prime editing efficiency according to the type of terminator in the prime editor expression cassette.
Figure 20 is a schematic diagram of a prime editor expression cassette comprising different promoters.
Figure 21 is a schematic diagram of recombinant vectors used for comparing prime editing efficiency according to the type of promoter in the prime editor expression cassette.
Figure 22 shows the results of analyzing prime editing efficiency according to the type of promoter in the prime editor expression cassette.
Figure 23 is a schematic diagram of prime editor constructs with different presence/absence, types, and arrangement order of RNA chaperone proteins.
Figure 24 is a schematic diagram of recombinant vectors used for comparing prime editing efficiency according to the presence/absence, type, and arrangement order of RNA chaperone proteins.
Figure 25 shows the results of analyzing prime editing efficiency according to the presence/absence, type, and arrangement order of RNA chaperone proteins.
Figure 26 shows the results of analyzing the types of edits at the target site based on the results of Figure 25.

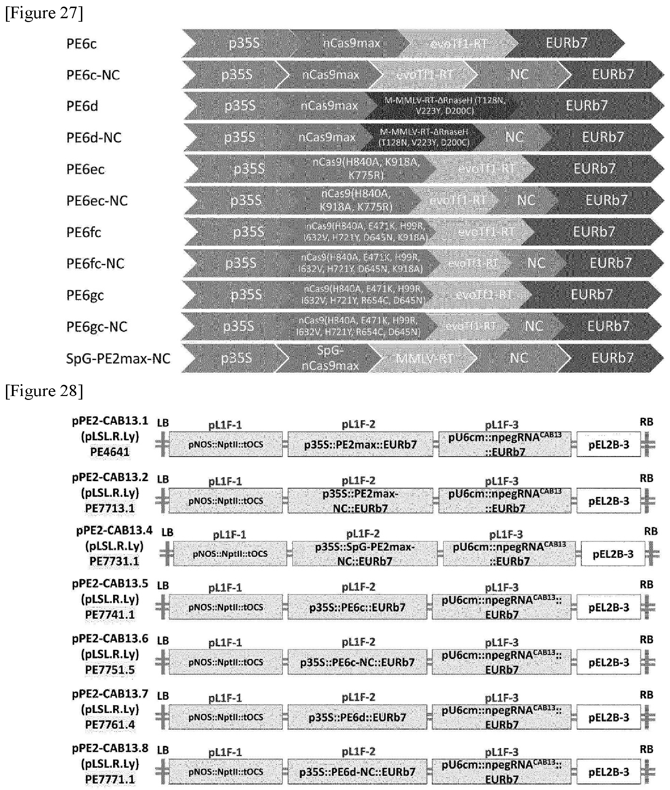

Figure 27 is a schematic diagram of a prime editor expression cassette PE6 comprising PE2max and different nickase Cas9 and/or reverse transcriptases. The coding sequences of PE6c, PE6c-NC, PE6d, PE6d-NC, PE6ec, PE6ec-NC, PE6fc, PE6fc-NC, PE6gc, and PE6gc-NC prime editors consist of SEQ ID NOs: 260 to 269, respectively, in sequence. nCas9max: mutant SpCas9 (R221K, N394K, D840A), SpG-nCas9max: mutant SpCas9 (R221K, N394K, H840A, D1135L, S1136W, G1218K, E1219Q, R1335Q, T1337R), evoTf1-RT: mutant Tf1 Retrotransposon reverse transcriptase (P70T, G72V, S87G, M102I, K106R, K118R, I128V, L158Q, F269L, A363V, K413E, S492N, S188K, I260L, S297Q, R288Q).
Figure 28 is a schematic diagram of recombinant vectors used for comparing prime editing efficiency according to the type of PE6 prime editor expression cassette.
Figure 29 shows the results of analyzing prime editing efficiency according to the type of PE6 prime editor expression cassette. PE2max was used as a comparative control group.
Figure 30 shows the results of comparing the prime editing efficiency of the PE2max-NC prime editor and the PE6c prime editor.
Figure 31 is a schematic diagram of recombinant vectors targeting six tomato genes used to examine the enhanced editing efficiency of the PE6c prime editor.
Figure 32 shows the results of analyzing the prime editing efficiency of the PE6c prime editor targeting six tomato genes, in which PE2max was used as a comparative control group.
Figure 33 is a schematic diagram of recombinant vectors comprising different PE6 prime editor expression cassettes targeting the *SlHKT1;2* gene.
Figure 34 shows the results of analyzing prime editing efficiency targeting the *SlHKT1;2* gene using various PE6 prime editors.
Figure 35 is a schematic diagram of recombinant vectors targeting *SlOR, SlCAB13*, and *SlCENH3(sub)* genes, comprising PE6c, PE6c-NC, or PE6ec-NC prime editor expression cassettes.
Figure 36 shows the results of analyzing prime editing efficiency of PE6c, PE6c-NC, and PE6ec-NC prime editors for three tomato genes, in which PE2max was used as a comparative control group.
Figure 37 shows the results of analyzing editing efficiency of PE2max-NC, PE6c, or PE6c-NC prime editors targeting the *AtPDS3* gene in Arabidopsis plants, including a schematic diagram of the recombinant vectors used and a graph of prime editing efficiency.
Figure 38 shows the results of analyzing editing efficiency of PE2max-NC or PE6c-NC prime editors targeting the *AtCENH3, AtOR*, and *AtALS* genes in Arabidopsis plants, including a schematic diagram of the recombinant vectors used and a table of analysis of the prime editing efficiency.

### BEST MODE(S) FOR CARRYING OUT THE INVENTION

In order to achieve the object of the invention, the present invention provides a recombinant vector for plant prime editing with enhanced editing efficiency, comprising a virus-based replicon, wherein the virus-based replicon comprises: a prime editing guide RNA (pegRNA) expression cassette regulated by a U6 composite promoter; and a prime editor expression cassette.

Prime editor is a type of gene editing system based on CRISPR-Cas9 that enables the introduction of genetic changes by cleaving only a single strand of DNA without inducing double-strand breaks. The prime editor may comprise a nickase Cas -reverse transcriptase (RT) fusion protein and a prime editing guide RNA (pegRNA), and in order to enhance the efficiency of the prime editing, additional domains or proteins may be further included in the fusion protein.

In the present invention, the term "pegRNA" comprises a guide sequence (or spacer sequence) that recognizes a target sequence, a tracrRNA (trans-activating CRISPR RNA) scaffold sequence, a primer binding site (PBS) required for initiation of reverse transcription, and a reverse transcription template (RTT) containing the desired genetic alteration. In addition, the pegRNA may include modified forms, such as a tevopreQ1 fusion, capable of improving the stability of the pegRNA.

In the pegRNA described above, the guide sequence refers to a sequence within the guide RNA that specifies the target site, and includes a sequence that is fully or partially complementary to the target sequence. The guide sequence is any polynucleotide sequence having complementarity to the target polynucleotide sequence that is sufficient to hybridize with the target DNA sequence and to direct sequence-specific binding of the gene editing complex to the target DNA sequence.

In the recombinant vector according to the present invention, the U6 composite promoter may comprise or consist of the nucleotide sequence of SEQ ID NO: 10, but is not limited thereto.

In the present invention, the term "replicon" refers to an autonomously controlled replication unit. The replication unit is a continuous DNA molecule in which replication is initiated at a specific site within the molecule, proceeds sequentially, and is terminated. Plasmids, viral DNA, and bacterial chromosomes each constitute a single replication unit.

In the recombinant vector according to the present invention, the virus-based replicon may be a replicon that is based on Bean Yellow Dwarf virus (BeYDV), Maize Streak virus (MSV), Tobacco mosaic virus (TMV), Cauliflower mosaic virus (CaMV), Tobacco ringspot virus (TRSV), Tobacco etch virus (TEV), Potato spindle tuber viroid (PSTVd), Cucumber mosaic virus (CMV), Papaya ringspot virus (PRSV), Tobacco streak virus (TSV), Pea enation mosaic virus (PEMV), Potato virus X (PVX), Potato virus Y (PVY), Potato leafroll virus (PLRV), Cowpea mosaic virus (CPMV), Bean common mosaic virus (BCMV), Beet curly top virus (BCTV), Alfalfa mosaic virus (AMV), Tomato spotted wilt virus (TSWV), Beet yellows virus (BYV), Cucumber green mottle mosaic virus (CGMMV), Turnip yellow mosaic virus (TYMV), Tobacco vein mottling virus (TVMV), Soybean mosaic virus (SMV), Rice tungro bacilliform virus (RTBV), Rice stripe virus (RSV), Rice dwarf virus (RDV), Maize streak virus (MSV), Maize chlorotic mottle virus (MCMV), Maize dwarf mosaic virus (MDMV), Maize chlorotic dwarf virus (MCDV), Barley stripe mosaic virus (BSMV), Wheat streak mosaic virus (WSMV), Wheat dwarf virus (WDV), or Wheat streak mosaic virus (WSMV), but not limited thereto. Preferably, it may be a replicon based on gemini virus such as BeYDV or MSV, but not limited thereto.

The replicon may include, linked in sequence, a long intergenic region (LIR); a promoter; a Rep/RepA protein coding sequence; a terminator; a short intergenic region (SIR); a multiple cloning site (MCS) into which a foreign gene to be expressed can be inserted; a SIR; and a LIR, but is not limited thereto. The LIR of the virus functions as an origin of replication and also as a promoter, and has the SIR functions similar to a terminator. With regard to the virus-based replicon vector, reference can be made to the applicant's previous work (Korean Patent Registration No. 2074744).

In the present invention, the virus-based replicon is inserted into T-DNA and introduced into plants via Agrobacterium. After delivery into the plant, it forms a circular replicon through rolling-circle replication and expresses the incorporated recombinant sequence.

In the recombinant vector according to the present invention, the replicon may further include a prime editor expression cassette and a selectable marker expression cassette.

In a recombinant vector according to one embodiment of the present invention, the prime editor may be a fusion protein 1 comprising a nickase Cas9 (CRISPR associated protein 9) protein, an MMLV (Moloney murine leukemia virus) reverse transcriptase, and a nucleocapsid protein; a fusion protein 2 comprising a nickase Cas9 protein and a Tf1 retrotransposon reverse transcriptase protein; or a fusion protein 3 comprising a nickase Cas9 protein, a Tf1 retrotransposon reverse transcriptase, and a nucleocapsid protein. The fusion protein 1 may be encoded by the nucleotide sequence of SEQ ID NO: 255, the fusion protein 2 may be encoded by the nucleotide sequence of SEQ ID NO: 260 or SEQ ID NO: 264, and the fusion protein 3 may be encoded by the nucleotide sequence of SEQ ID NO: 261 or SEQ ID NO: 265, but they are not limited thereto.

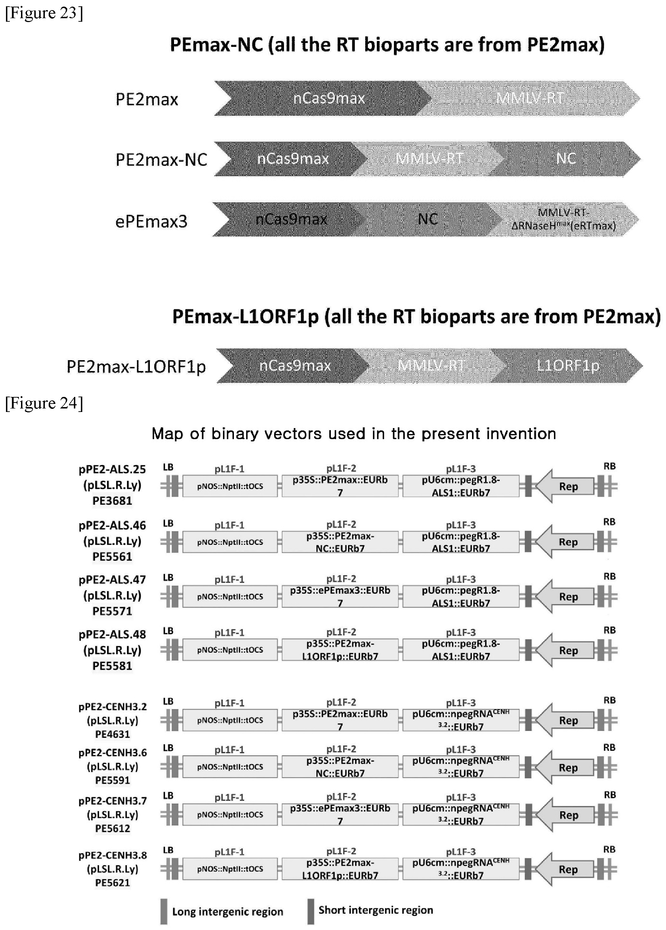

In one embodiment of the present invention, the prime editor encoded by the nucleotide sequence of SEQ ID NO: 255 is PE2max-NC, the prime editor encoded by the nucleotide sequence of SEQ ID NO: 260 is PE6c, the prime editor encoded by the nucleotide sequence of SEQ ID NO: 264 is PE6ec, the prime editor encoded by the nucleotide sequence of SEQ ID NO: 261 is PE6c-NC, and the prime editor encoded by the nucleotide sequence of SEQ ID NO: 265 is PE6ec-NC (see Figures 23 and 27).

In another embodiment of the present invention, the nickase Cas9 protein of the PE2max-NC, PE6c, and PE6c-NC prime editors may be a SpCas9 (*Streptococcus pyogenes* Cas9) protein having R221K, N394K, and D840A mutations, and the nickase Cas9 protein of the PE6ec and PE6ec-NC prime editors may be a SpCas9 protein having K775R, H840A, and K918A mutations. However, the present invention is not limited thereto, and a nickase Cas9 protein having R221K, N394K, H840A, D1135L, S1136W, G1218K, E1219Q, R1335Q, and T1337R mutations (SpG-nCas9max, encoded by the nucleotide sequence of SEQ ID NO: 278) may also be used.

In addition, the MMLV reverse transcriptase of the PE2max-NC prime editor may have D200N, T306K, W313F, T330P, and L603W mutations, and the Tf1 retrotransposon reverse transcriptase of the PE6c, PE6ec, PE6c-NC, and PE6ec-NC prime editors may have P70T, G72V, S87G, M102I, K106R, K118R, I128V, L158Q, F269L, A363V, K413E, S492N, S188K, I260L, S297Q, and R288Q mutations, but they are not limited thereto.

The prime editor expression cassette according to one embodiment of the present invention may be regulated by a CaMV 35S promoter and an EURb7 terminator (EU + Rb7 dual terminator), but is not limited thereto.

The prime editor according to the present invention is characterized by exhibiting superior prime editing efficiency in plants compared to prime editors composed of other combinations of elements.

In addition, in the recombinant vector according to the present invention, the selectable marker is used to select cells transformed with the introduced vector, and markers that confer selectable phenotypes such as drug resistance, nutritional auxotrophy, resistance to cytotoxic agents, or expression of surface proteins may be used. In an environment treated with a selective agent, only cells expressing the selection marker survive, thereby enabling selection of transformed cells.

In the present invention, the term "recombinant" refers to a cell that replicates heterologous nucleic acids, expresses said nucleic acids, or expresses peptides, heterologous peptides, or proteins encoded by heterologous nucleic acids. A recombinant cell may express a gene or gene fragment that is not found in the natural form of the cell, in either sense or antisense orientation. In addition, a recombinant cell may express a gene found in a natural state cell, wherein the gene is modified and has been artificially reintroduced into the cell by artificial means.

The term "vector" is used to refer to DNA fragment(s) or nucleic acid molecules that are delivered into cells. A vector can replicate DNA and reproduce independently in a host cell. The term "carrier" is often used interchangeably with "vector."

In the present invention, the term "expression cassette" refers to a nucleic acid sequence comprising one or more genes and regulatory sequences controlling their expression, for example, any combination of cis-acting transcriptional regulatory elements. The expression cassette of the present invention includes three main elements: i) a promoter; ii) a second polynucleotide operably linked to the promoter and referred to as a "coding polynucleotide" or "coding sequence" (also referred to as a coding gene), whose transcription is directed by the promoter when the expression cassette is introduced into a cell; and iii) a terminator polynucleotide (also referred to as a transcription terminator) that directs termination of transcription and is located immediately downstream of the second polynucleotide.

In the present invention, the term "promoter" refers to a region of DNA upstream of a structural gene to which RNA polymerase binds to initiate transcription. A "plant promoter" is a promoter capable of initiating transcription in plant cells. A "constitutive promoter" is a promoter that is active under most environmental conditions and developmental stages or cellular differentiation states. Since selection of transformants may be performed at various stages and in various tissues, a constitutive promoter may be preferred in the present invention. Accordingly, a constitutive promoter does not limit the selection capability.

In the present invention, the promoters include promoters suitable for transformation, and are preferably, but not limited to, a CaMV 35S promoter, a U6-26 core promoter, a U6 composite promoter, an actin promoter, a ubiquitin promoter, a pEMU promoter, a MAS promoter, or a histone promoter.

In the present invention, a terminator may be a conventional terminator, and examples thereof include, but are not limited to, an EURb7 (EU + Rb7 double terminator), a t3T (EU + 35S + Rb7 triple terminator), a nopaline synthase (NOS) terminator, a rice α-amylase RAmy1 A terminator, a phaseolin terminator, and a terminator of the octopine synthase gene from *Agrobacterium tumefaciens*.

The present invention further provides a method for enhancing prime editing efficiency in plants, comprising transforming one or more plant cells with the recombinant vector of the present invention.

In the method for enhancing prime editing efficiency in plants according to the present invention, the recombinant vector is as described above.

Plant transformation refers to any method of transferring DNA into a plant. Such transformation methods do not necessarily require a regeneration and/or tissue culture period. Plant transformation is now commonly applied not only to dicotyledonous plants but also to monocotyledonous plant species. In principle, any transformation method can be used to introduce the recombinant DNA according to the present invention into suitable progenitor cells. Such methods include the calcium/polyethylene glycol method for protoplasts (Krens, F.A. et al., 1982, Nature 296, 72-74; Negrutiu I. et al., 1987, Plant Mol. Biol. 8, 363-373), electroporation of protoplasts (Shillito R.D. et al., 1985, Bio/Technol. 3, 1099-1102), microinjection into plant material (Crossway A. et al., 1986, Mol. Gen. Genet. 202, 179-185), particle bombardment of various plant materials (DNA- or RNA-coated) (Klein T.M. et al., 1987, Nature 327, 70), Agrobacterium tumefaciens-mediated gene transfer, infection with (defective) viruses (EP 0 301 316), and transformation of plant tissues such as infiltration or mature pollen or microspores. A preferred method according to the present invention includes Agrobacterium-mediated DNA delivery.

In the method according to one embodiment of the present invention, the transformation may be mediated by an *Agrobacterium tumefaciens* strain having the genetic background of EHA105 or EHA105 superagro ver.2, but is not limited thereto.

The transformation is mediated by an *Agrobacterium tumefaciens* strain, and plant tissues may be treated at a temperature of 30-35°C, preferably 30-32°C, and more preferably 31°C after co-cultivation with *Agrobacterium tumefaciens*, but is not limited thereto.

The "plant cells" used for plant transformation may be any plant cells. Plant cells include cultured cells, cultured tissues, cultured organs, or whole plants. "Plant tissue" includes differentiated or undifferentiated plant tissues, such as, but not limited to, roots, stems, leaves, pollen, seeds, tumor tissues, and various forms of cells used in culture, including single cells, protoplasts, shoots, and callus tissues. Plant tissues may be in planta or in an organ culture, tissue culture, or cell culture state.

In the method of the present invention, transformed plant cells may be regenerated into whole plants. The method for regenerating transformed plants from the transformed plant cells may use any method known in the art. Techniques for regeneration of mature plants from callus or protoplast cultures are well known in the art for numerous species.

The present invention still further provides a composition for enhancing prime editing efficiency in plants comprising the recombinant vector of the present invention, as an active ingredient.

In the composition according to the present invention, the recombinant vector is as described above. The composition of the present invention is characterized in that it enhances prime editing efficiency at target sites in plants.

Hereinbelow, the present invention will be described in greater detail with reference to Examples. However, those Examples are provided solely to illustrate the present invention, and it is evident that the scope of the present invention is not limited by those Examples.

### EXAMPLES

### Experimental Methods

### 1. Combination of PE protein components and plasmid construction

For production of the PE proteins used in the present invention, nCas9 (H840A) from PE2max, PE4max, and ePEmax1, and nCas9 and eRT from ePEmax2 and ePEmax3 were cloned from the plasmid pCMV-PEmax-P2A-hMLH1dn (Addgene #174828). The nCas9 of PPE-NC-v1 was cloned from the nCas9-PPE plasmid (Addgene #140445), and NC and RT were cloned from the pH-ePPE plasmid (Addgene #183097). ePPE was cloned from the pH-ePPE plasmid, and the NC sequence used in ePEmax1, ePEmax2, ePEmax3, and PE2max-NC was also cloned from the pH-ePPE plasmid.

For the basic PE expression cassette, transcription was controlled using a CaMV 35S promoter (p35S) (Addgene #50267) and an EU + Rb7 double terminator (EURB7) (Diamos and Mason, 2018, Plant Biotechnol J. 16:1971-1982). In experiments evaluating the effect of promoters, various promoters (p35S, double CaMV 35S promoter (p2x35S) (Addgene #50269), and tomato EF1α (PSLEF1α) (Niu et al., Plant Biotechnol J. 2023, 21:5-7)) were used together with the EU + 35S + Rb7 triple terminator (t3T). To evaluate the effect of terminators on PE efficiency, p35S was combined with various terminators (t35S, tNOS, EURB7, and t3T).

The modified epegRNA was designed by introducing fundamental modifications into the scaffold of the epegRNA based on an altered SpCas9 scaffold (Nelson et al., Nat Biotechnol. 2022, 40:402-410). For transcription of pegRNA, either a U6-26 core promoter (pU6-26 core) or a U6 composite promoter (Jiang et al., Genome Biol. 2020, 21:257) was used. Information regarding edited sites/loci, gRNA, and RTT is provided in detail in Table 1. The pegRNA was amplified by PCR, cloned into an expression cassette, and then assembled into a binary vector by Golden Gate assembly.

For T-DNA-based PE tools, a selection marker (NPTII, Addgene #51144), a PE protein, and a pegRNA expression cassette were cloned into the pAGM4723 plasmid (Addgene #48015). In cases using a geminivirus replication system, the expression cassette was cloned into the previously reported pLSL.R.Ly vector (Vu et al., Plant Biotechnol J. 2020, 18:2133-2143). All biological parts [pNOS::NptII::tOCS (from pICSL11024, Addgene Plasmid #51144), PE2max (SEQ ID NO: 1), PE4max (SEQ ID NO: 2), PPE-NC-v1 (SEQ ID NO: 3), ePPE (SEQ ID NO: 4), ePEmax1 (SEQ ID NO: 5), ePEmax2 (SEQ ID NO: 6), PE2max-NC (SEQ ID NO: 255), ePEmax3 (SEQ ID NO: 256), 35S promoter (SEQ ID NO: 7), U6-26 core promoter (SEQ ID NO: 8), U6 composite promoter (SEQ ID NO: 9), EU + Rb7 double terminator (EURb7; SEQ ID NO: 10), EU + 35S + Rb7 triple terminator (t3T; SEQ ID NO: 11)] were domesticated into MoClo level 0 plasmids (Weber et al., 2011, PLoS One 6(2):e16765) and assembled into PE protein combinations and binary vectors.

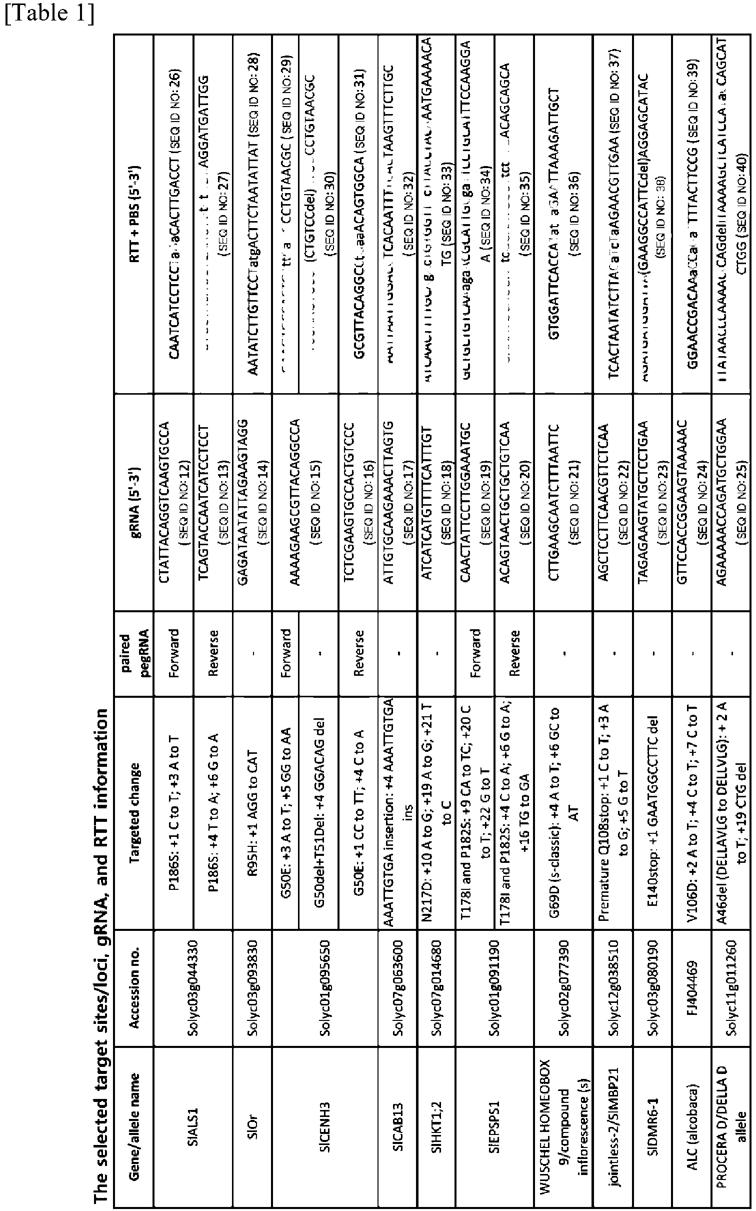

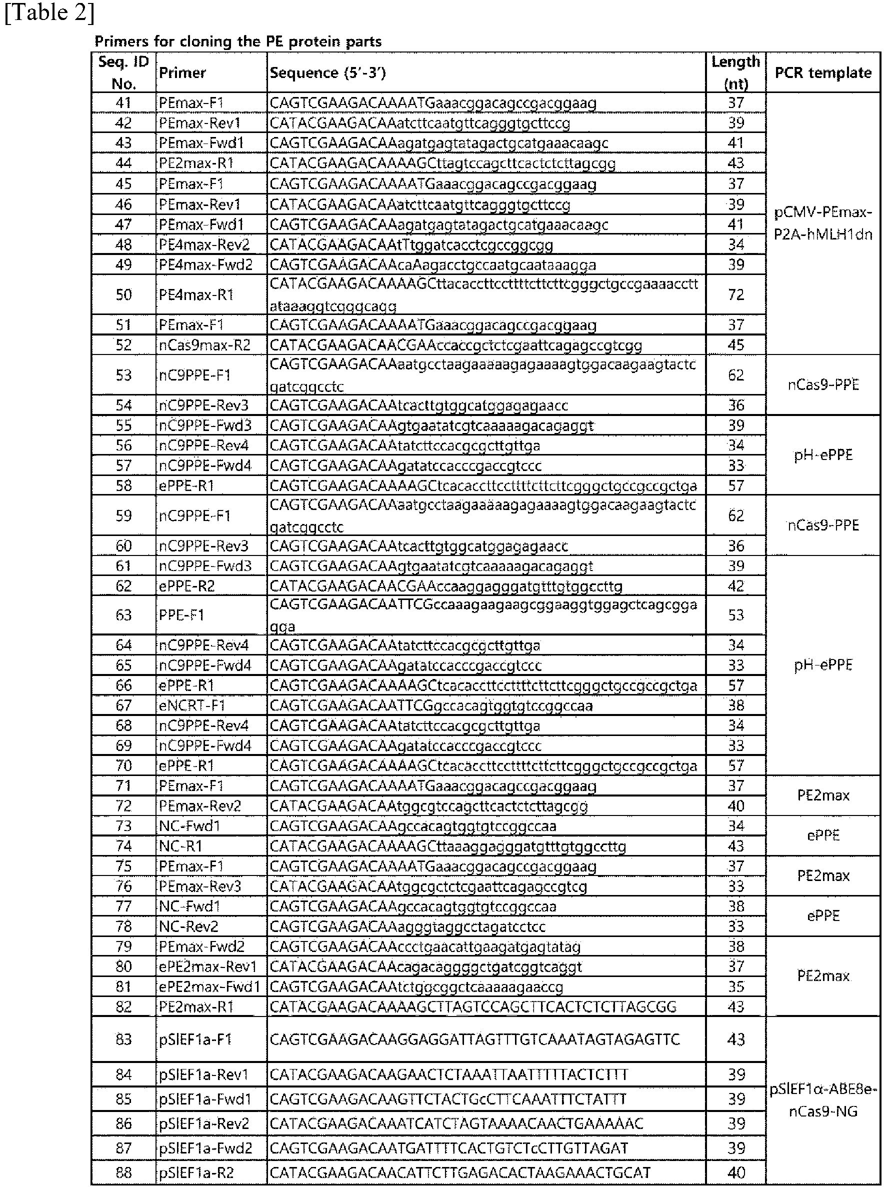

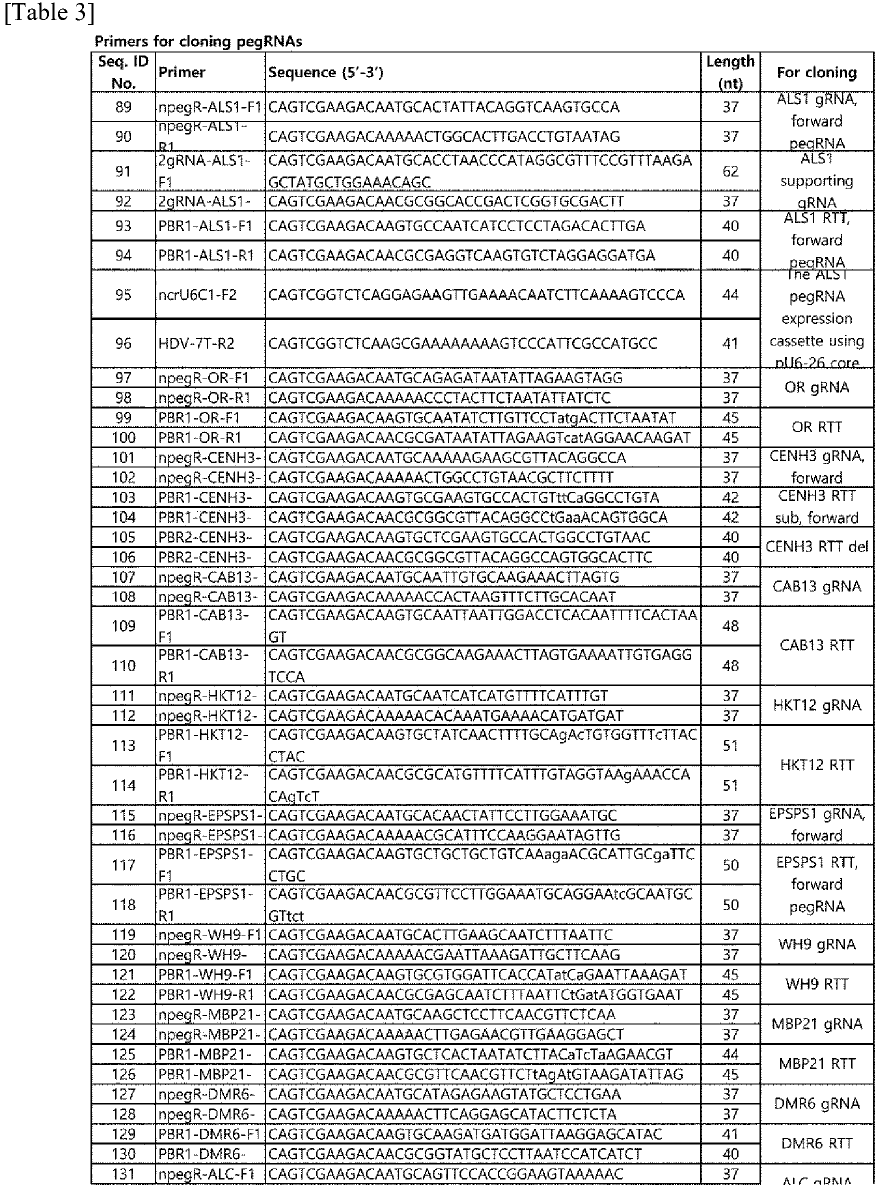

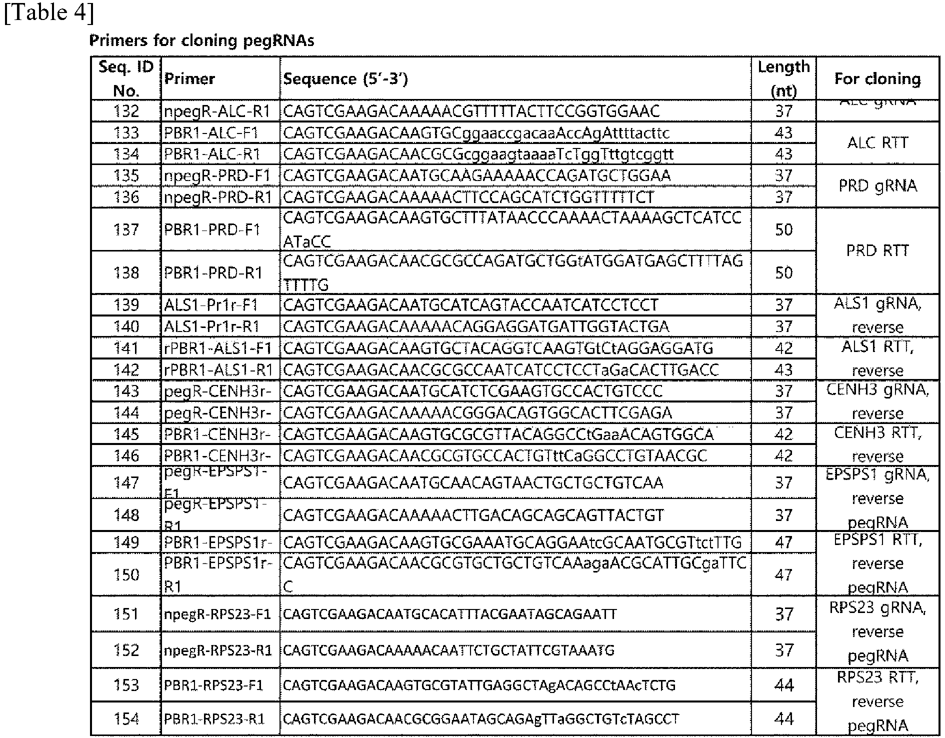

### 2. Agrobacterium-mediated tomato transformation and analysis of prime editing efficiency

Agrobacterium-mediated transformation of tomato was performed as described by Vu et al. In the present invention, the *Agrobacterium tumefaciens* strains GV3101::pMP90, EHA105, and Super Agrobacterium version 2 based on EHA105 (Nonaka et al., 2019, Front Plant Sci. 10:1204) were used. Cotyledons from 7-day-old tomato seedlings were excised and used for transformation. Agrobacteria carrying PE plasmids were cultured, harvested by centrifugation, and resuspended in ABM-MS solution (Vu et al., 2020) supplemented with 100 µM acetosyringone to an OD₆₀₀ₙₘ of 0.8. Agrobacteria were activated by incubation at 28°C for 1 hour prior to transformation. Agrobacteria and cotyledons were co-incubated at room temperature for 20 minutes, and the transformed plant tissues were co-cultivated for 2 days before washing and transfer to selection medium. Samples were cultured at 31°C for 5 days, followed by 5 days at 28°C, and subsequently maintained at 25°C for the remaining stages. Regenerated shoots were selected on medium containing 80 mg/L kanamycin and induced for rooting prior to transfer to soil. Hardened plants were then used for evaluation of PE efficiency. For temperature treatment experiments, after co-cultivation, explants were cultured at different temperatures for 5 days, followed by 5 days at 28°C, and then transferred to 25°C for later stages.

To evaluate PE efficiency, samples were collected at 16 days post-transformation (dpt) followed by targeted deep sequencing. At all plant growth stages, leaves from transformants were collected and screened for PE alleles using PCR and Sanger sequencing.

### 3. Targeted deep sequencing

Genomic DNA (gDNA) was extracted from cotyledons or plant leaves using the CTAB method. For analysis, a MiniSeq sequencing service (MiniSeqTM System, Illumina, USA) was used. MiniSeq samples were prepared through three rounds of PCR using primers listed in Tables 5 and 6. The third PCR was performed using primers provided by the manufacturer to assign sample IDs. Subsequently, raw MiniSeq sequencing data FASTQ files were analyzed using Cas-Analyzer (Park et al., 2016, Bioinformatics 33:286-288) and CRISPResso2 (Clement et al., 2019, Nat Biotechnol. 37:224-226) with the parameters listed in Tables 7 and 8.

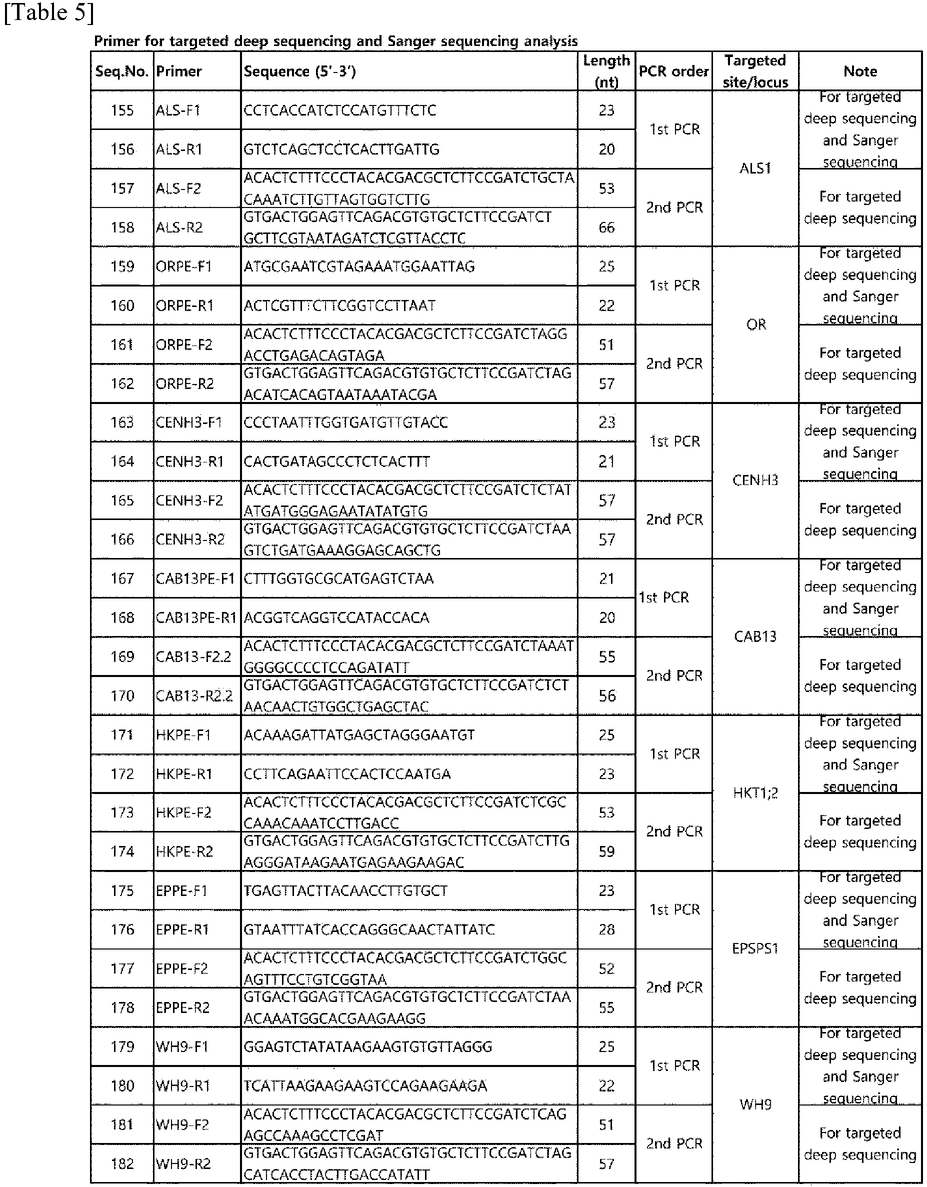

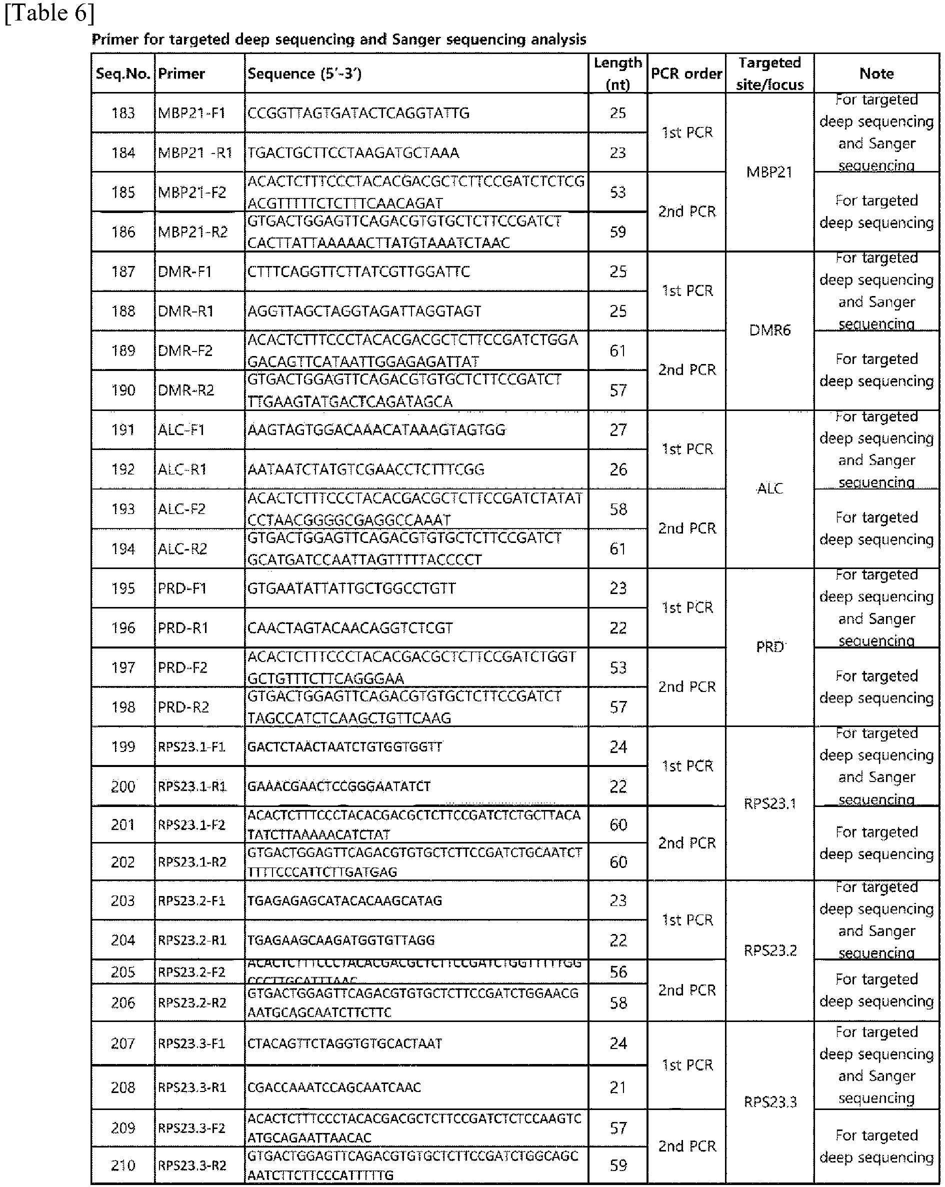

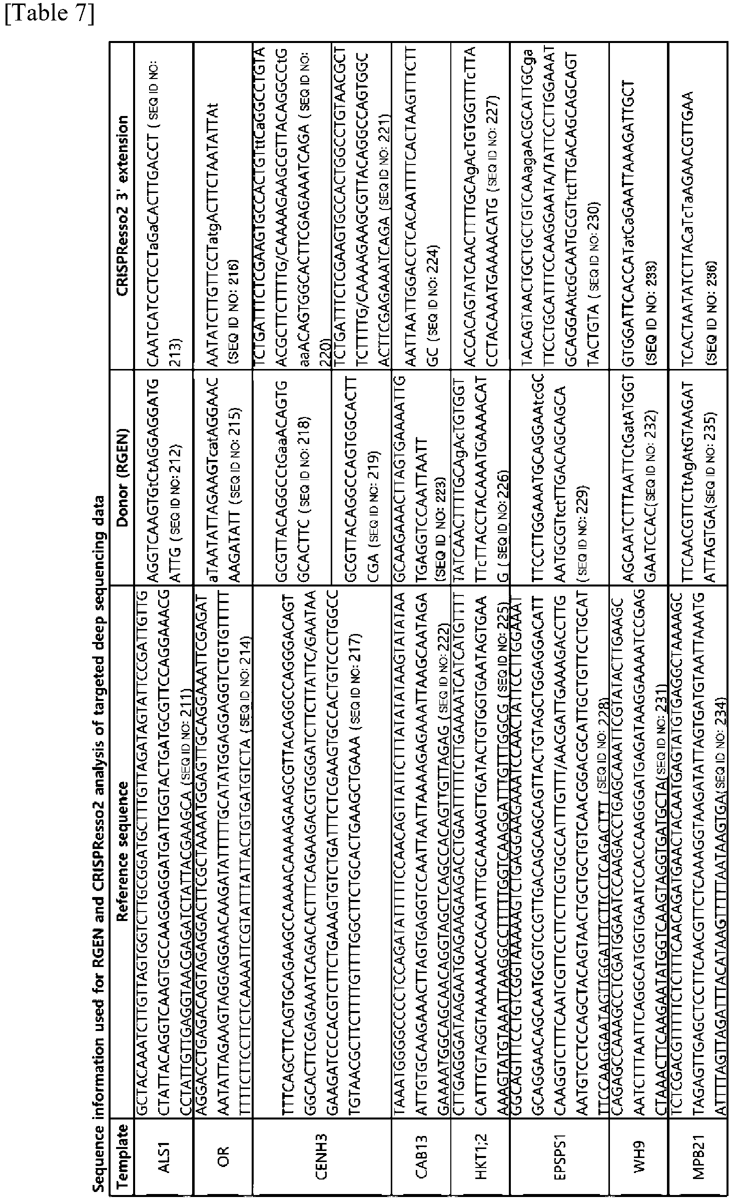

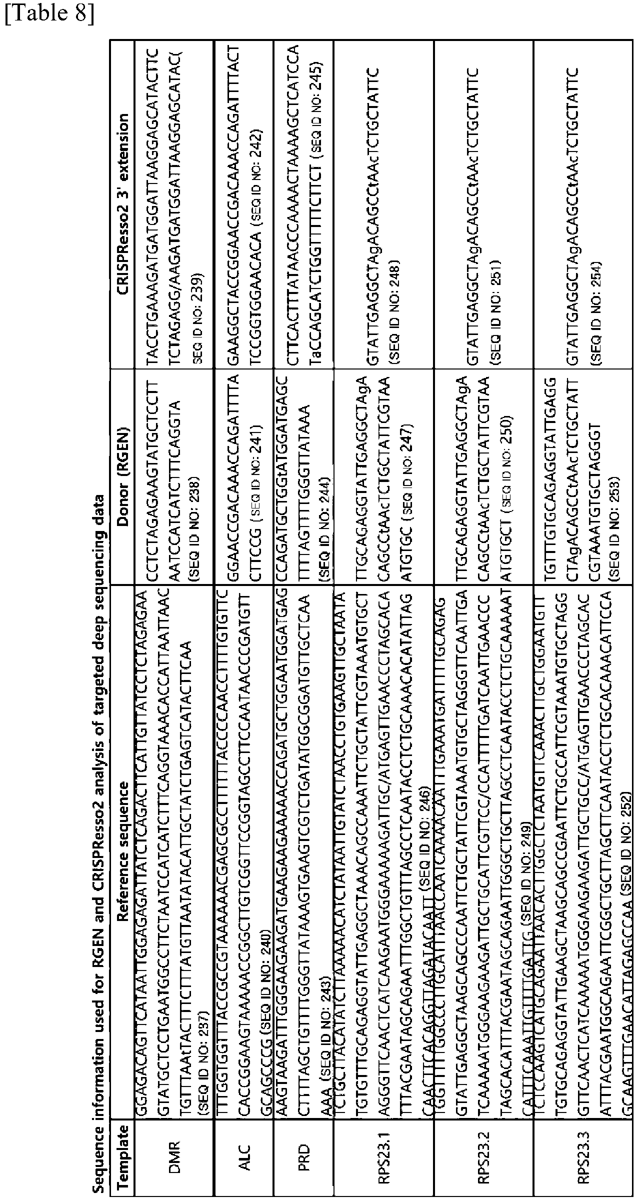

### 4. Determination of transformants containing PE alleles

The PE alleles in the transformed plants were identified after the hardening phase. To screen for the PE alleles, three different leaf segments from each plant were collected and combined to isolate gDNA using the CTAB method. PCR amplification was conducted to amplify the DNA sequences flanking the target region, and the PCR products were sequenced using the Sanger sequencing method. The resulting sequencing chromatograms were analyzed using the ICE Synthgo tool to identify potential events carrying the PE alleles. Some representative PE events were further examined through targeted deep sequencing.

### 5. Determination of presence or absence of T-DNA and replicons in PE events

To determine the presence or absence of T-DNA and replicon, the gDNA of the PE plants was used as a template for PCR reactions, employing primer pairs specified for the right border (RB) of T-DNA and the circular form of the replicon. The replicon primers were specifically designed to amplify only the circularized DNA that had been released from the vector (Vu et al., 2020). The PCR products were analyzed on a 1% agarose gel, and the presence or absence of T-DNA and replicon was determined by determining the expected band sizes corresponding to T-DNA and replicon, respectively.

### 6. Analysis of off-targets

The gRNA sequence of the pegRNA was used to search for potential off-target sites in the tomato genome database *Solanum lycopersicum* (SL2.4), in which the number of mismatches with the gRNA sequence was less than four, using the Cas-OFinder tool. The 16-day post-transformation (dpt) cotyledon explants, transformed with pegRNA containing the identified potential off-target sites, were analyzed by targeted deep sequencing using the primers listed in Tables 5 and 6.

### 7. Data analysis

All experiments were conducted in at least triplicate. The editing results, statistical analysis, and scatter plots were processed using MS Excel and GraphPad Prism 9.0 software. Multiple comparisons were conducted using the uncorrected Fisher's LSD test.

### Example 1. Analysis of Editing Efficiency Based on Prime Editor Protein Combinations and pegRNA Transcription Regulatory Promoter Types

Inventors of the present invention constructed various recombinant vectors for prime editors targeting the tomato *ALS1* gene (Figures 2 and 3). These vectors were then used to transform cotyledons of the tomato Hongkwang cultivar via Agrobacterium-mediated transformation. Afterward, nucleotide change was analyzed by Next-Generation Sequencing (NGS) of the target site.

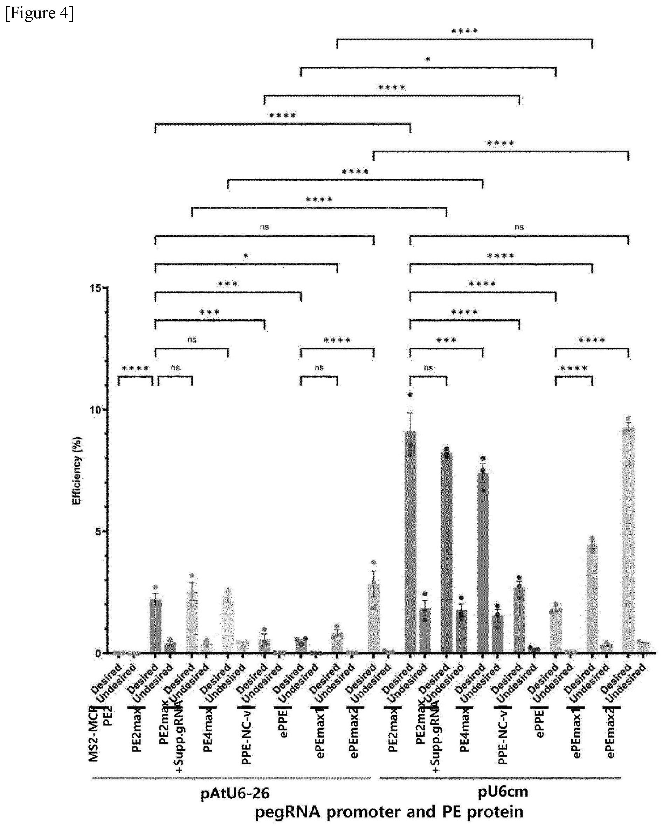

As shown in Figure 4, it was found that the editing efficiency was significantly higher in the experimental group in which prime editing was performed with vectors driven by the U6 composite promoter for pegRNA transcription, compared to the vector driven by the U6-26 core promoter for pegRNA expression. Among them, especially in the groups using the PE2max, PE2max + Supp.gRNA, and ePEmax2 prime editors, the highest amount of desired base correction was observed.

After regenerating plants from the calluses in which the tomato *ALS1* gene was edited, the desired prime editing was examined again in the regenerated plants. As a result, similar to the efficiency observed in the calluses, excellent frequency and efficiency were found in the edited plants using the PE2max, PE2max + Supp.gRNA, and ePEmax2 prime editors (Figure 5). Furthermore, genomic DNA was extracted from the regenerated plants, and PCR analysis was performed to check for the presence of T-DNA and replicons. It was consequently found that in some PE0 event plants, neither T-DNA nor replicons were detected (Figure 6).

### Example 2. Analysis of Prime Editing Efficiency Based on Transformation-Mediating Strain

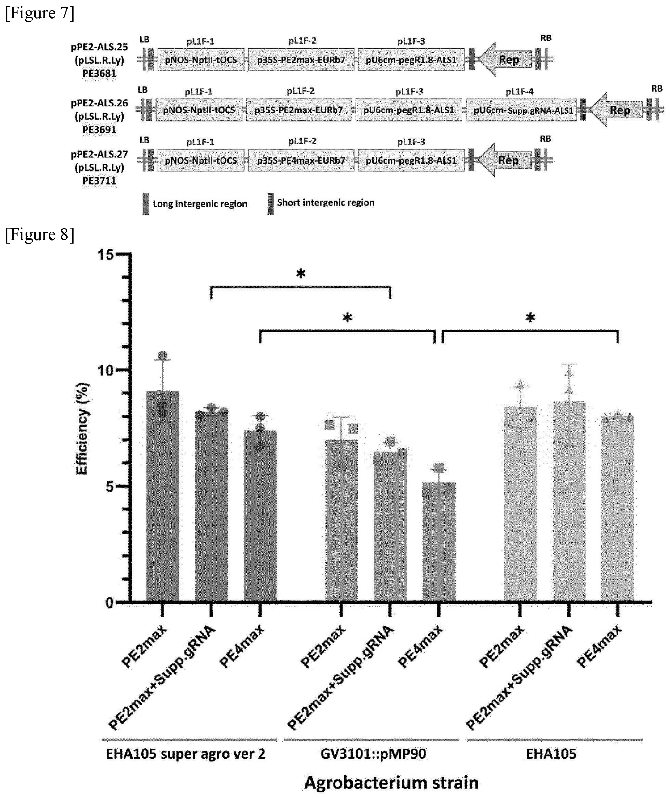

Inventors of the present invention analyzed the prime editing efficiency depending on the Agrobacterium strain by constructing recombinant vectors using PE2max, PE2max + Supp.gRNA, or PE4max (Figure 7). Each of these vectors was then introduced into Agrobacterium strains EHA105 superagro ver.2 (EHA105sv2), GV3101::pMP90, or EHA105, and the prime editing efficiency was analyzed.

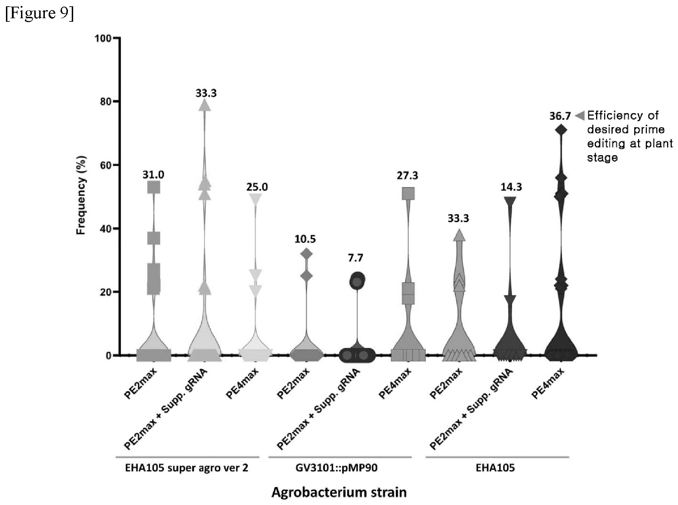

When comparing the prime editing efficiency at the callus stage through NGS analysis, as shown in Figure 8, it was found that the editing efficiency using the EHA105sv2 and EHA105 strains was 1.27 to 1.43 times and 1.2 to 1.55 times higher, respectively, compared to the GV3101::pMP90 strain. Similarly, when comparing the frequency and efficiency of prime editing in regenerated plants, the EHA105sv2 and EHA105 strains showed 0.92 to 4.33 times and 1.34 to 3.17 times better efficiency, respectively, compared to the GV3101::pMP90 strain (Figure 9).

### Example 3. Analysis of Prime Editing Efficiency Based on Delivery Method of Prime Editor Tools

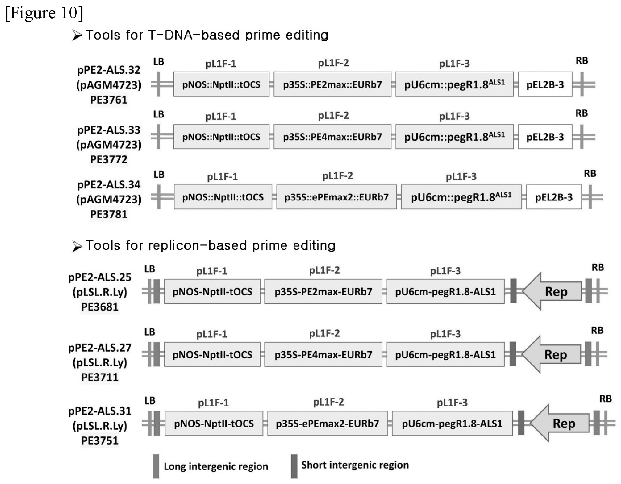

Inventors of the present invention compared the prime editing efficiency based on the delivery method of the prime editor tool, specifically focusing on T-DNA versus geminivirus-based replicon systems. The same components were cloned into either T-DNA or replicon-based recombinant vectors (Figure 10), and these vectors were then introduced into tomato plants using the *Agrobacterium* EHA105sv2 strain to evaluate the prime editing efficiency.

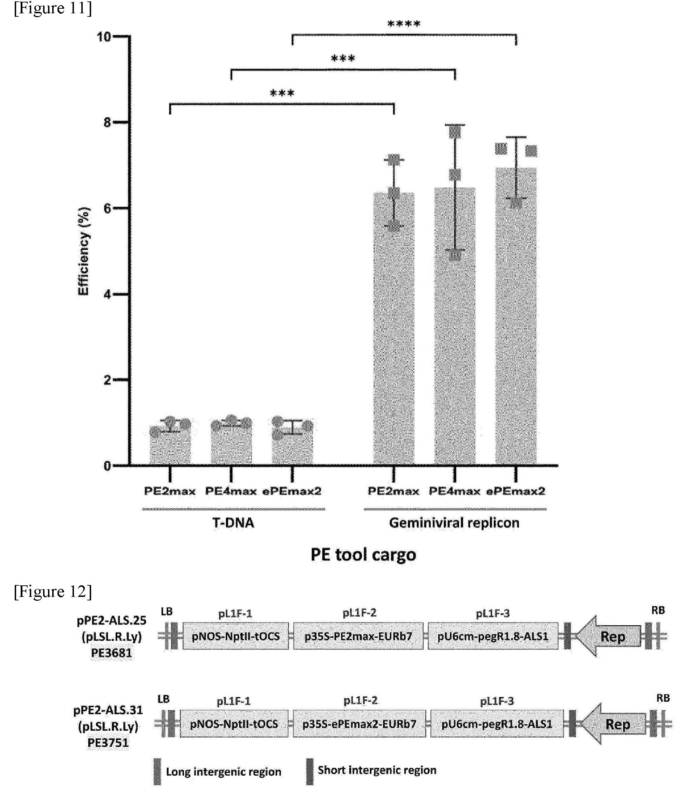

When comparing the prime editing efficiency at the callus stage using NGS analysis, it was found that the geminivirus-based replicon system achieved a prime editing efficiency that was 6.55 to 7.79 times higher than that using the T-DNA vector system, as it is illustrated in Figure 11.

### Example 4. Analysis of Prime Editing Efficiency Based on Temperature Conditions for Prime Editing

In this example, the prime editing efficiency was evaluated based on the treatment temperature (25, 28, 31, and 34°C) of plant tissues within 5 days after co-cultivation with Agrobacterium. Specifically, recombinant vectors based on geminivirus replicons, including PE2max or ePEmax2 prime editors (Figure 12), were used for transformation into tomato plants via *Agrobacterium* EHA105sv2 strain, followed by analysis of the prime editing efficiency.

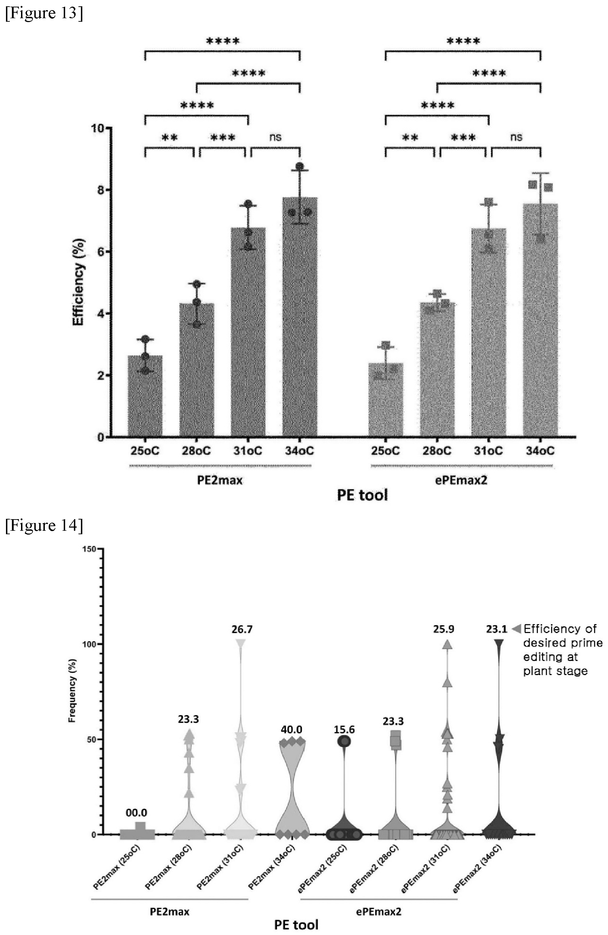

The results showed that at the callus stage, the prime editing efficiency increased in proportion to the treatment temperature (Figure 13). Additionally, when analyzing the frequency and efficiency of prime editing in the regenerated plants, it was found that the highest prime editing frequency in PE2max prime-edited plants occurred at 31°C, while for ePEmax2 prime-edited plants, the highest frequency was observed at both 31°C and 34°C (Figure 14). Therefore, considering both the prime editing efficiency and regeneration of plants, it was recognized that the optimal temperature condition for prime editing is 31°C.

### Example 5. Comparison of Prime Editing Efficiency Across Various Target Genes

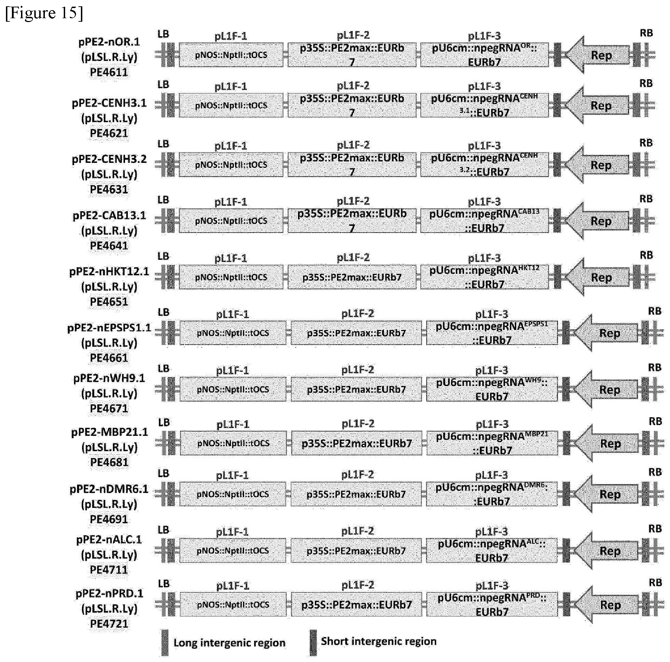

Inventors of the present invention conducted prime editing on different target gene loci in tomato and compared the prime editing efficiency across these target genes. The schematic diagram of the replicon-based vectors used in this embodiment is shown in Figure 15. The transformation into tomato plants was carried out via *Agrobacterium* EHA105sv2 strain, followed by analysis of the prime editing efficiency.

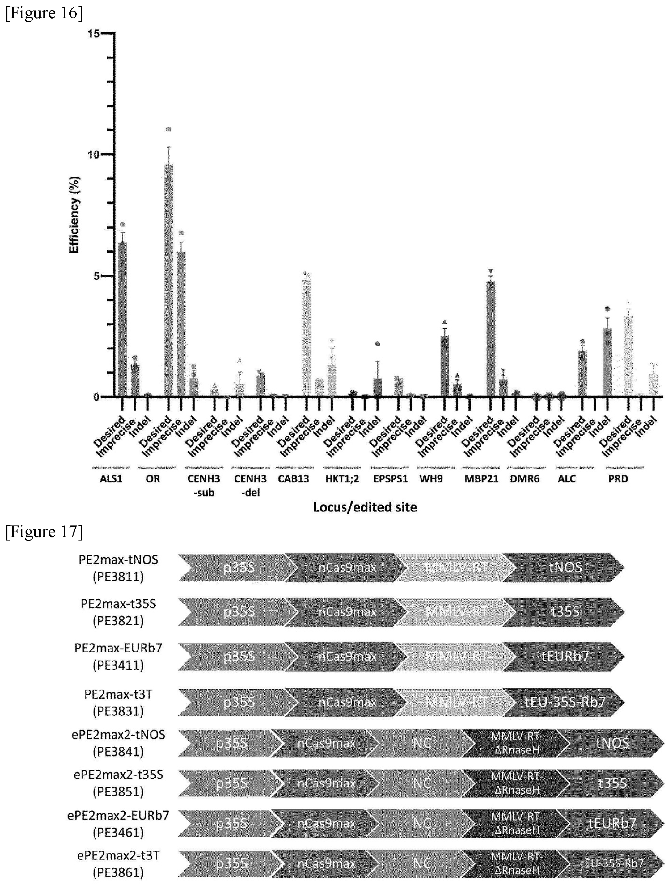

The results, as shown in Figure 16, revealed that there were differences in prime editing efficiency among the various target genes. Out of the 11 genes tested, 7 showed editing efficiencies of 2% or higher, while 4 genes had editing efficiencies below 1%. For the *DMR6* gene, no prime editing occurred. These results indicate that prime editing can be applied to various positions and loci in the tomato genome.

### Example 6. Comparison of Prime Editing Efficiency Based on Terminator Types in Prime Editor Expression Cassettes

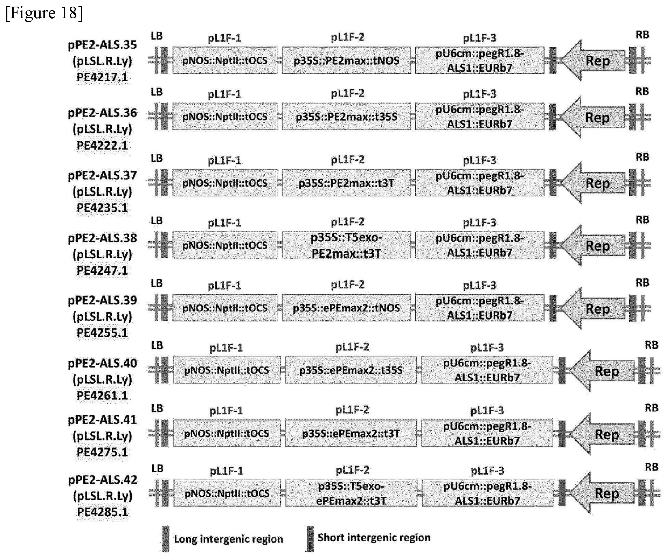

To analyze the differences in prime editing efficiency based on the type of terminator included in the prime editor protein expression cassette, the prime editor protein expression cassettes were designed as shown in Figure 17, and replicon-based recombinant vectors were constructed as shown in Figure 18. These vectors were then introduced into tomato plants via the *Agrobacterium* EHA105sv2 strain, followed by analysis of the prime editing efficiency.

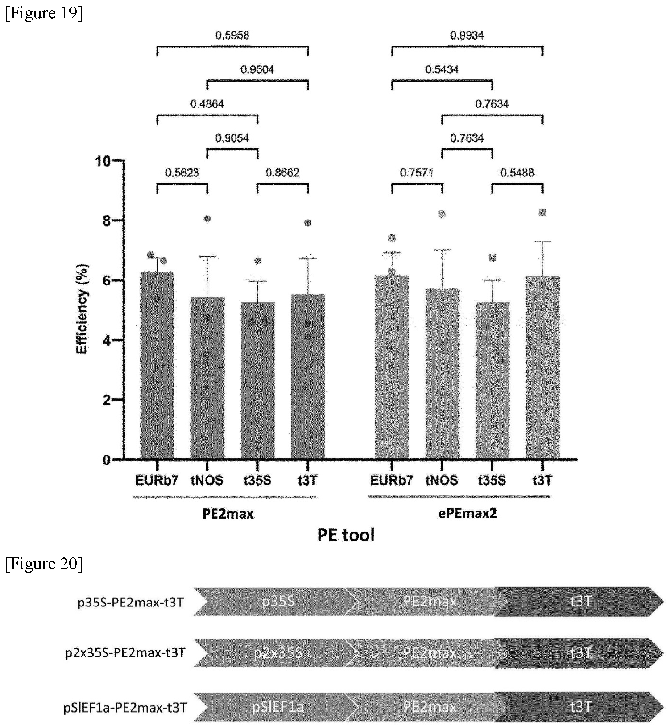

The results showed that the t35S terminator exhibited the lowest efficiency, but no significant differences in efficiency were observed among the different terminator types (Figure 19).

### Example 7. Comparison of Prime Editing Efficiency Based on Promoter Types in Prime Editor Expression Cassettes

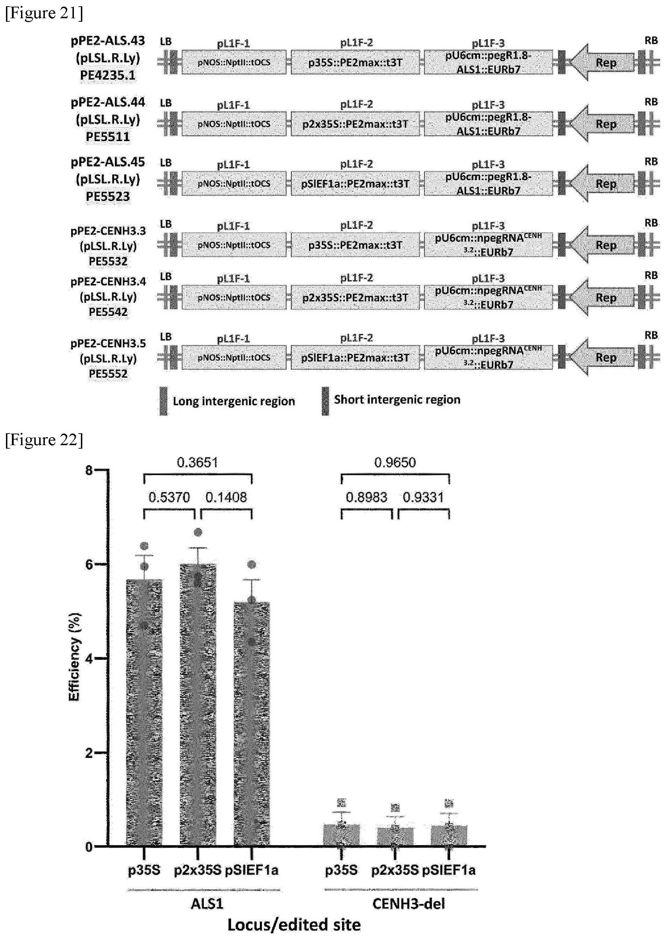

In this experiment, to analyze the differences in prime editing efficiency based on the type of promoter regulating the transcription of the prime editor protein, prime editor protein expression cassettes were designed as shown in Figure 20, and replicon-based recombinant vectors were constructed as shown in Figure 21. These vectors were then introduced into tomato plants via the *Agrobacterium* EHA105sv2 strain, followed by analysis of the prime editing efficiency.

The results showed that the pSlEF1 promoter exhibited the lowest efficiency, but no significant differences in efficiency were observed among the different promoter types (Figure 22).

### Example 8. Comparison of Prime Editing Efficiency Based on RNA Chaperone

In this experiment, the differences in prime editing efficiency based on the presence, type, or sequence arrangement of RNA chaperone proteins within the prime editor structure were analyzed. Using PE2max, which showed superior prime editing efficiency in Example 1, as the basic structure, recombinant vectors were constructed by adding NC (nucleocapsid) or L1ORF1p to the prime editor, or by altering the sequence order of these components within the prime editor (Figures 23 and 24).

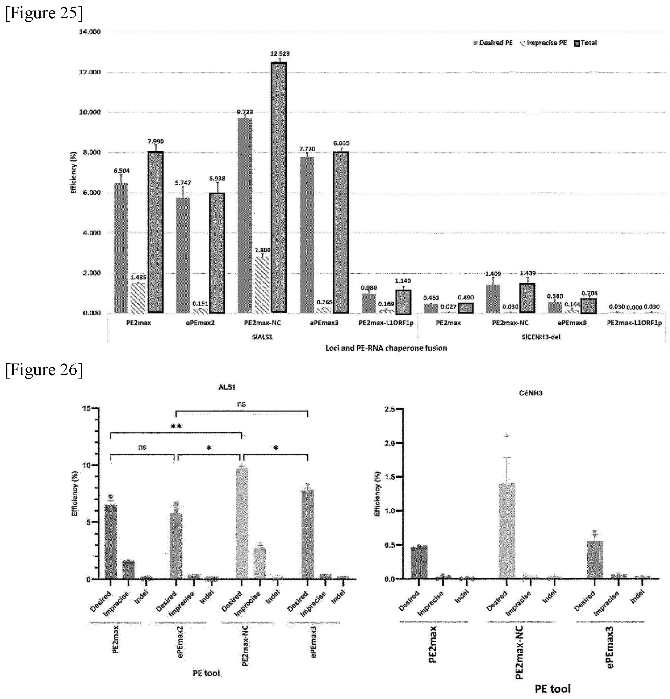

After transforming tomato plants with these recombinant vectors through *Agrobacterium* EHA105sv2 strain, the prime editing efficiency was analyzed. As a result, the overall editing efficiency was found to be highest in the experimental group using the PE2max-NC prime editor (Figure 25). Additionally, the efficiency of desired prime editing was also highest in the group using the PE2max-NC prime editor (Figure 26).

These results indicate that the use of a prime editor fused with nCas9max-MMLV RT-NC (nucleocapsid) significantly enhances prime editing efficiency in plants.

### Example 9. Analysis of Editing Efficiency Based on Changes in Prime Editor Protein Composition

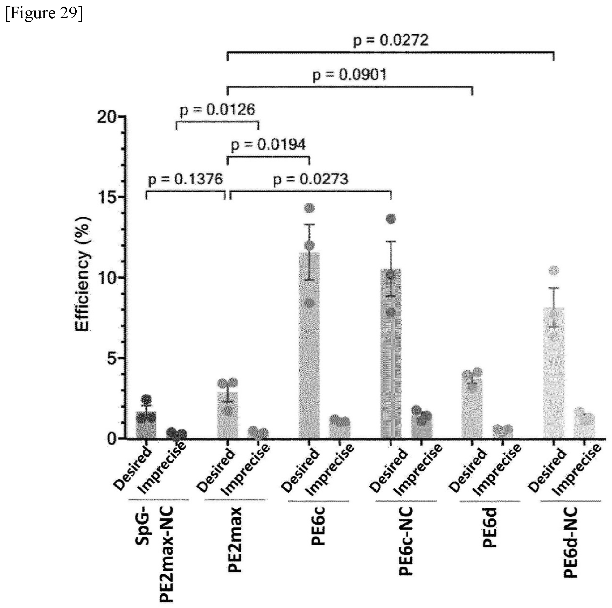

In this experiment, novel prime editor expression cassettes were created by modifying the nickase Cas9 protein, reverse transcriptase, or both used in PE2max or PE2max-NC prime editors (Figure 27). Subsequently, recombinant vectors targeting the *SlCAB13* gene, which contains these expression cassettes, were constructed (Figure 28). The recombinant vectors were then introduced into tomato plants via *Agrobacterium* EHA105sv2 strain for prime editing efficiency analysis. The results showed that the experimental groups using the PE6c and PE6c-NC prime editors exhibited higher editing efficiencies compared to the experimental group using the PE2max prime editor (Figure 29). Additionally, the editing efficiency was enhanced in the experimental group using the PE6d-NC prime editor compared to the group using the PE6d prime editor.

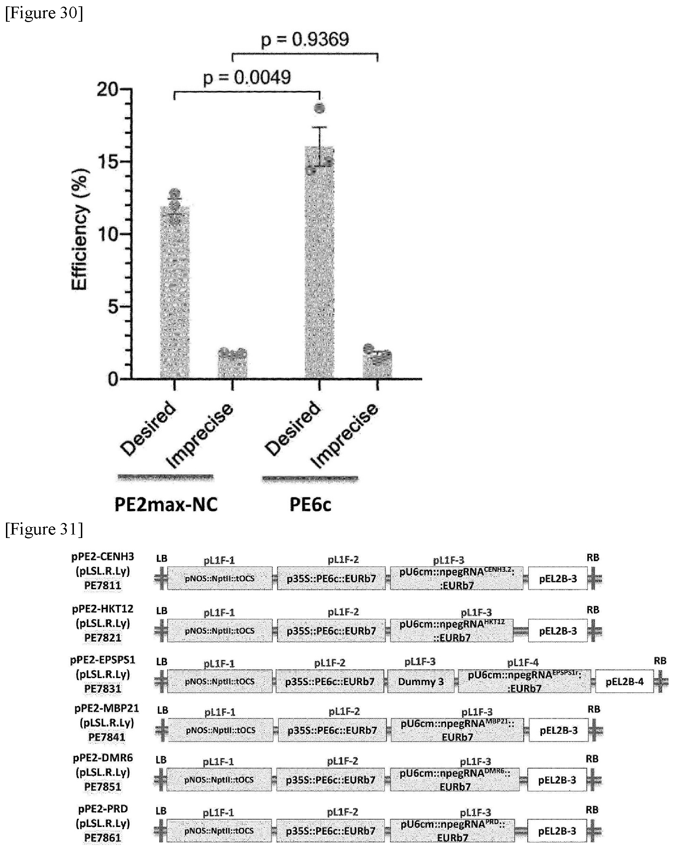

As a result of comparing the editing efficiency of the PE2max-NC prime editor, which showed higher editing efficiency than the PE2max prime editor in Example 8, and that of the PE6c prime editor, it was found that the experiment using the PE6c prime editor exhibited significantly higher editing efficiency, particularly with an increase in the desired prime editing, as shown in Figure 30.

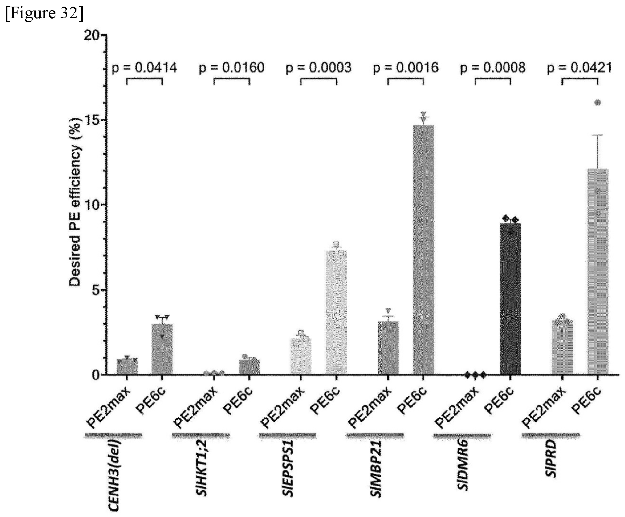

To examine the enhanced prime editing efficiency of the PE6c prime editor, the inventors of the present invention constructed recombinant vectors targeting genes other than the *SlCAB13* gene in tomato plants (Figure 31) and analyzed the prime editing efficiency, using the PE2max prime editor as the control. The results, shown in Figure 32, indicated that the PE6c prime editor exhibited higher prime editing efficiency than the PE2max prime editor for all six tested genes, with the *SlHKT1;2* gene showing an 8.9-fold increase in editing efficiency.

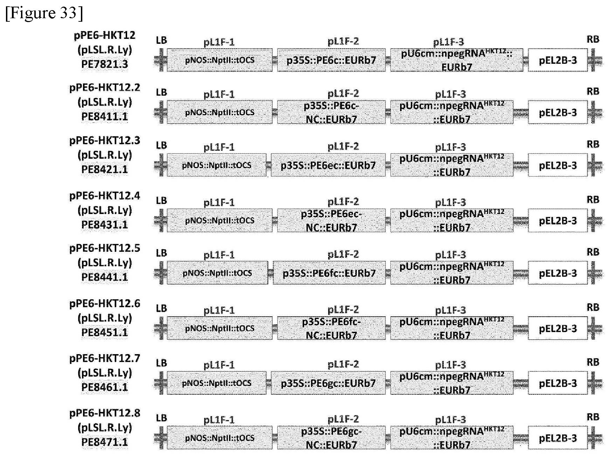

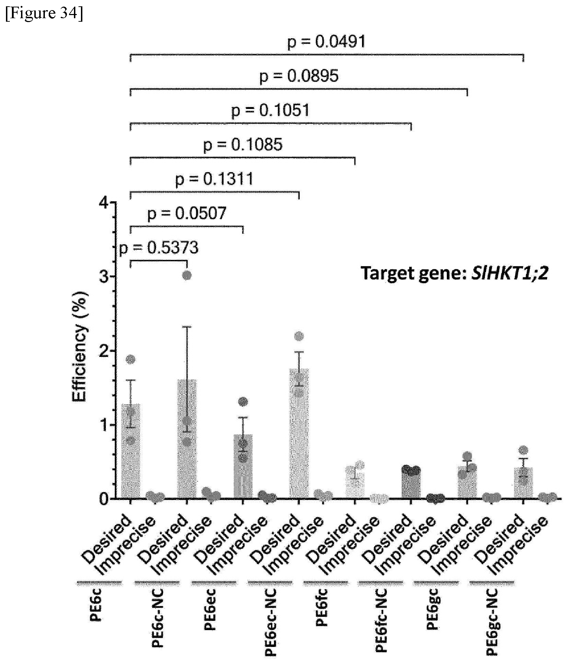

A recombinant vector containing various PE6 prime editor expression cassettes (see Figure 27) was constructed targeting the *SlHKT1;2* gene (Figure 33), and the prime editing efficiency was analyzed. The results showed that the PE6c-NC and PE6ec-NC prime editors exhibited approximately 1.5% higher prime editing efficiency compared to the PE6c prime editor (see Figure 34).

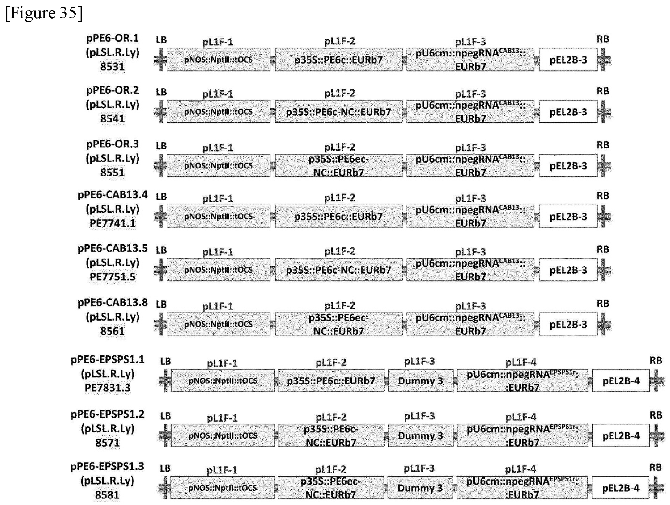

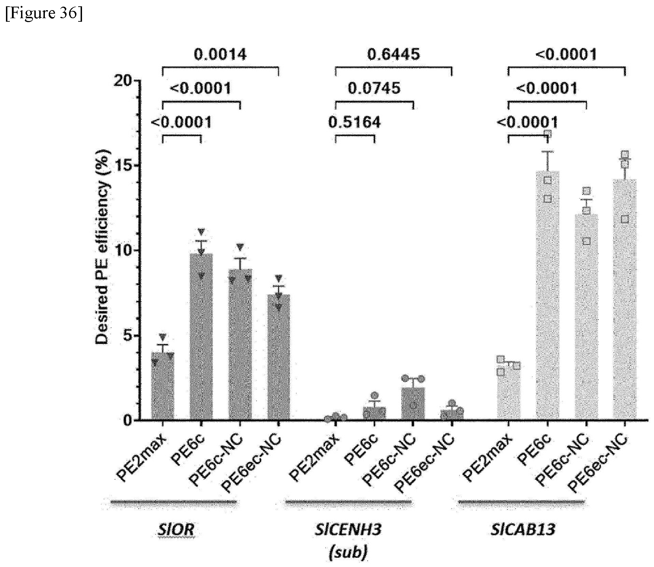

Inventors of the present invention then constructed recombinant vectors targeting the *SlOR, SlCAB13*, and *SlCENH3(sub)* genes, containing PE6c, PE6c-NC, and PE6ec-NC prime editor expression cassettes (see Figure 35), and analyzed the prime editing efficiency. The three PE6 prime editors showed higher prime editing efficiency than the PE2max prime editor. Except for the *SlCENH3(sub)* gene, the PE6c prime editor demonstrated the highest efficiency, with the prime editing efficiency for the *SlCAB13* gene reaching 14.68% (see Figure 36).

### Example 10. Prime Editing Efficiency Analysis in Arabidopsis thaliana

In this experiment, the editing efficiency of PE2max-NC, PE6c, or PE6c-NC prime editors in *Arabidopsis thaliana* plants was analyzed. The target gene was *AtPDS3*, and the recombinant vectors, constructed as described in previous Examples, were introduced into the plants via *Agrobacterium* EHA105sv2 strain for transformation.

**[Table 9]**

| **The selected target sites/loci, gRNA, and RTT information for *Arabidopsis*** | | | | | |
|---|---|---|---|---|---|
| **No.** | **Gene/allele name** | **Accession no.** | **Targeted change** | **gRNA (5'-3')** | **RTT (5'-3')** |
| 1 | *AtALS* | AT3G48560 | P197S: +3 A to t; +5+6 GG to AA | CTGTACCAATCATACGACGA (SEQ ID NO: 270) | TCACAGGACAAGTttCaCGTCGTATGATTC (SEQ ID NO: 271) |
| 2 | *AtOR* | AT5G61670 | R90H: +3+4 TC to AT; +6 GtoA | GCAAGAAGATCTTGTTTCGT (SEQ ID NO: 272) | AGATAACATTCGAAGtCatCGAAACAAGATCT (SEQ ID NO: 273) |
| 3 | *AtCENH3* | AT1G01370 | G83T84 del: +5 GGAACC del | AAAAGAAGCGTTACAGGCCA (SEQ ID NO: 274) | TCTCTTTTAGAGCAACTGGCCTGTATCGAT (SEQ ID NO: 275) |
| 4 | *AtPDS3* | AT4G14210 | M30stop: +2 AATG to TTGA | ATCTGGAGGTTGTGAACTAA (SEQ ID NO: 276) | TAAAGCTATGTCCtcaaAGTTCACAACC (SEQ ID NO: 277) |

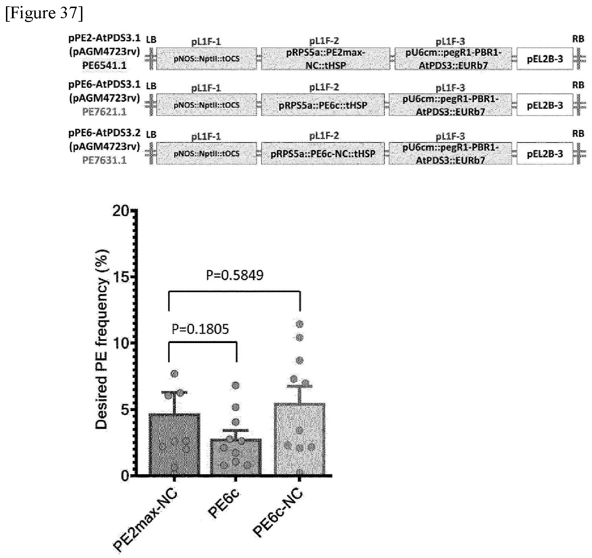

The analysis revealed that the prime editing efficiency for the *AtPDS3* gene was higher with PE2max-NC and PE6c-NC prime editors compared to the PE6c prime editor, and the editing efficiency of PE2max-NC and PE6c-NC was similar (Figure 37).

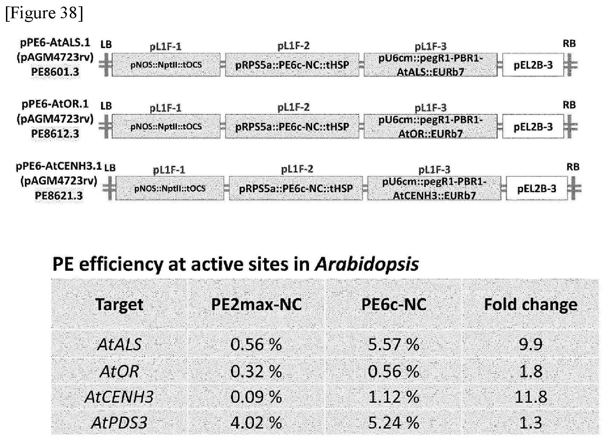

Inventors of the present invention further analyzed prime editing efficiency targeting the *AtCENH3, AtOR*, and *AtALS* genes using the PE2max-NC and PE6c-NC prime editors. As a result, it was found that the PE6c-NC prime editor exhibited higher prime editing efficiency at the *AtALS* and *AtCENH3* genes (Figure 38).

These results suggest that PE2max-NC, PE6c, PE6c-NC, PE6ec, or PE6ec-NC prime editors can be advantageously used to have enhanced prime editing efficiency in dicotyledonous plants.

## Claims

1. A recombinant vector for plant prime editing with enhanced editing efficiency, comprising a virus-based replicon, wherein the virus-based replicon comprises: a prime editing guide RNA (pegRNA) expression cassette regulated by a U6 composite promoter; and a prime editor expression cassette.

2. The recombinant vector according to claim 1, wherein the virus is selected from the group consisting of Bean Yellow Dwarf virus (BeYDV), Maize Streak virus (MSV), Tobacco mosaic virus (TMV), Cauliflower mosaic virus (CaMV), Tobacco ringspot virus (TRSV), Tobacco etch virus (TEV), Potato spindle tuber viroid (PSTVd), Cucumber mosaic virus (CMV), Papaya ringspot virus (PRSV), Tobacco streak virus (TSV), Pea enation mosaic virus (PEMV), Potato virus X (PVX), Potato virus Y (PVY), Potato leafroll virus (PLRV), Cowpea mosaic virus (CPMV), Bean common mosaic virus (BCMV), Beet curly top virus (BCTV), Alfalfa mosaic virus (AMV), Tomato spotted wilt virus (TSWV), Beet yellows virus (BYV), Cucumber green mottle mosaic virus (CGMMV), Turnip yellow mosaic virus (TYMV), Tobacco vein mottling virus (TVMV), Soybean mosaic virus (SMV), Rice tungro bacilliform virus (RTBV), Rice stripe virus (RSV), Rice dwarf virus (RDV), Maize chlorotic mottle virus (MCMV), Maize dwarf mosaic virus (MDMV), Maize chlorotic dwarf virus (MCDV), Barley stripe mosaic virus (BSMV), Wheat dwarf virus (WDV), and Wheat streak mosaic virus (WSMV).

3. The recombinant vector according to claim 1, wherein the U6 composite promoter comprises the nucleotide sequence of SEQ ID NO: 10.

4. The recombinant vector according to claim 1, wherein expression of the prime editor expression cassette is regulated by a CaMV 35S promoter and an EURb7 terminator.

5. The recombinant vector according to claim 1, wherein the prime editor is a fusion protein 1 comprising a nickase Cas9 (CRISPR associated protein 9) protein, an MMLV (Moloney murine leukemia virus) reverse transcriptase, and a nucleocapsid protein; a fusion protein 2 comprising a nickase Cas9 protein and a Tf1 retrotransposon reverse transcriptase protein; or a fusion protein 3 comprising a nickase Cas9 protein, a Tf1 retrotransposon reverse transcriptase, and a nucleocapsid protein.

6. The recombinant vector according to claim 5, wherein the fusion protein 1 is encoded by the nucleotide sequence of SEQ ID NO: 255, the fusion protein 2 is encoded by the nucleotide sequence of SEQ ID NO: 260 or SEQ ID NO: 264, and the fusion protein 3 is encoded by the nucleotide sequence of SEQ ID NO: 261 or SEQ ID NO: 265.

7. The recombinant vector according to claim 1, wherein the replicon further includes a selectable marker expression cassette.

8. A method for enhancing prime editing efficiency in plants, comprising transforming one or more plant cells with the recombinant vector according to any one of claims 1 to 7.

9. The method according to claim 8, wherein the transformation is mediated by an *Agrobacterium tumefaciens* strain having the genetic background of EHA105 or EHA105 superagro ver.2.

10. The method according to claim 8, wherein the transformation is mediated by an *Agrobacterium tumefaciens* strain, and plant tissues are treated at a temperature of 30 to 35°C after co-cultivation with *Agrobacterium tumefaciens*.

11. A composition for enhancing prime editing efficiency in plants comprising the recombinant vector according to any one of claims 1 to 7 as an active ingredient.
